# FASCICULE DE BREVET EUROPEEN

(11) **EP 3 749 637 B1**
(45) Date de publication et mention de la délivrance du brevet: **10.11.2021**
(21) Numéro de dépôt: 19710041.5
(22) Date de dépôt: 07.02.2019
(51) Int. Cl.: C07C 217/58, C07C 217/84, C07C 265/14, C07C 251/48, C07C 247/24, C08G 59/50, C08G 59/32, C08G 18/76

(54) **COMPOSES BIPHENYLE DIFONCTIONNELS, PREPARATION ET UTILISATIONS**
DIFUNKTIONELLE BIPHENYLVERBINDUNGEN, HERSTELLUNG UND VERWENDUNGEN
DIFUNCTIONAL BIPHENYL COMPOUNDS, PREPARATION, AND USES

(30) Priorité: 08.02.2018 FR 1851071
(43) Date de publication de la demande: 16.12.2020
(73) Titulaire: ArianeGroup SAS, 75015 Paris (FR); Centre National de la Recherche Scientifique, 75016 Paris (FR)
(72) Inventeur: SAVONNET, Etienne, 75018 PARIS (FR); DEFOORT, Brigitte, 33160 SAINT-MEDARD-EN-JALLES (FR); CRAMAIL, Henri, 33350 SAINTE-TERRE (FR); GRELIER, Stéphane, 40160 PARENTIS-EN-BORN (FR); GRAU, Etienne, 33000 BORDEAUX (FR)
(74) Mandataire: Cabinet Beau de Loménie
(86) Numéro de dépôt international: PCT/FR2019/050273
(87) Numéro de publication internationale: WO 2019/155169

(56) Documents cités:
- EP-A1- 0 352 775
- FR-A1- 2 734 832
- R F CHAPMAN ET AL: "Studies related to the Chemistry of Melanins. Part VII. Attempts to Synthesise Hydroxylated Bi-indolyls, Biphenyls, and Indoline-2-carboxylic Acid as Possible Intermediates in the Formation of Melanins from 3,4-Dihydroxyphenethylamine and 3,4-Dihydroxyphenylalanine", JOURNAL OF THE CHEMICAL SOCIETY C: ORGANIC, vol. 1970, no. 6, 1 janvier 1970 (1970-01-01), pages 865-872, XP055195932, DOI: 10.1039/j39700000865
- GAUR M ET AL: "Improved device performance based on crosslinking of poly (3-hexylthiophene)", SYNTHETIC METALS, ELSEVIER SEQUOIA, LAUSANNE, CH, vol. 160, no. 19-20, 1 octobre 2010 (2010-10-01), pages 2061-2064, XP027380392, ISSN: 0379-6779 [extrait le 2010-08-21]
- KURT MAURER ET AL: "Neue Abkömmlinge des Dihydro-divanillins und Erfahrungen über die katalytische Reduktion von Nitrostyrolen", JOURNAL FÜR PRAKTISCHE CHEMIE : PRACTICAL APPLICATIONS AND APPLIED CHEMISTRY : COVERING ALL ASPECTS OF APPLIED CHEMISTRY, vol. 144, no. 1-2, 15 octobre 1935 (1935-10-15), pages 41-48, XP055526673, DE ISSN: 0021-8383, DOI: 10.1002/prac.19351440103
- AUDREY LLEVOT ET AL: "Renewable (semi)aromatic polyesters from symmetrical vanillin-based dimers", POLYMER CHEMISTRY, vol. 6, no. 33, 3 juillet 2015 (2015-07-03), pages 6058-6066, XP055235410, GB ISSN: 1759-9954, DOI: 10.1039/C5PY00824G

## Description

La présente invention a pour principal objet des composés biphényle difonctionnels, i.e. des composés biphényle avec deux fonctions réactives. Lesdites deux fonctions réactives sont de type méthylamino, isocyanate ou amino (voir la formule (I) donnée ci-après). Ces composés conviennent tout particulièrement comme durcisseurs (agents de polymérisation réticulante ou encore agents de réticulation difonctionnels, de par la présence des deux fonctions réactives dans leur formule), de monomères ou prépolymères thermodurcissables, notamment :
- dans un contexte de fonctions réactives avec groupe amino (de type méthylamino ou amino), de monomères ou prépolymères thermodurcissables de type résine époxy (à fonctions époxy), polycarbonate (à fonctions carbonate) et polyacide carboxylique (à fonctions acide carboxylique) pour, respectivement, obtenir des polyépoxydes, des poly(hydroxy)uréthanes (« NiPU : Non-isocyanate PolyUrethane ») et des polyamides ; et
- dans un contexte de fonctions réactives avec groupe isocyanate (fonctions réactives de type isocyanate), de monomères ou prépolymères thermodurcissables de type résine polyol et résine polyamine pour, respectivement, obtenir des polyuréthanes et des polyamides.

Ces composés sont particulièrement intéressants en ce qu'ils conviennent parfaitement pour l'obtention de produits (type monomères ou prépolymères thermodurcissables thermodurcis), présentant de hautes propriétés mécaniques et de tenue en température (convenant à de nombreuses applications, notamment dans le domaine des adhésifs et des composites) et en ce que, pour nombre d'entre eux, ils peuvent être obtenus, outre par des voies de synthèse conventionnelles (connues dans le domaine de la pétrochimie), à partir de la biomasse (à partir de la lignine, plus précisément à partir de la vanilline) (on peut parler de composés biosourcés).

La présente invention a également pour objet la préparation desdits composés biphényle difonctionnels et leurs utilisations.

A ce jour :
particulièrement pour la polymérisation réticulante des résines époxy, tout particulièrement pour la polymérisation réticulante de celles les plus utilisées, obtenues à partir du monomère (n = 0 (voir la formule ci-dessous)) ou prépolymère (n≠0 (voir la formule ci-dessous)) diglycidyléther de bisphénol-A (DiGlycydyl Ether de Bisphénol-A = DGEBA), qui présente la formule ci-après : 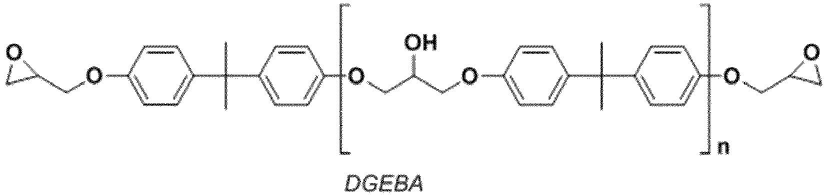 on utilise la plupart du temps des durcisseurs conventionnels avec fonctions (réactives) de type amine. On peut citer, de façon nullement limitative, le diaminodiphényl sulfone (DDS), le diaminodiphénylméthane ((DDM) ou méthylène dianiline (MDA)), l'isophorone diamine (IPDA), le dicyandiamide, la 4,4-méthylène-bis(2-isopropyl-6-méthylaniline) (notamment commercialisée par Loza Ltd, sous la dénomination commerciale Lonzacure^{®} M-MIPA), et la 4,4'-méthylène-bis(2,6-diisopropylamineaniline (notamment commercialisée par Loza Ltd, sous la dénomination commerciale Lonzacure^{®} M-DIPA). A toutes fins utiles, on montre ci-après les formules des deux premiers durcisseurs conventionnels de l'art antérieur identifiés dans la liste ci-dessus :

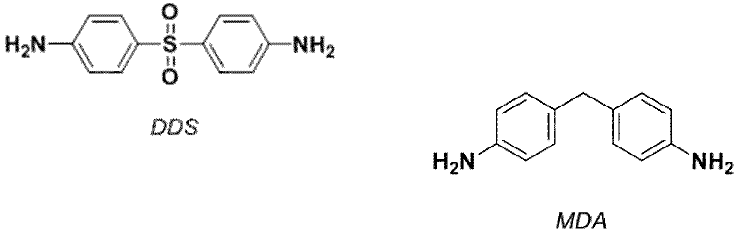

Notons incidemment que peuvent aussi être utilisés et sont aussi utilisés, comme durcisseur de résines époxy, des alcools, des acides carboxyliques, des anhydrides, des thiols et même des isocyanates (tous, on l'a compris, difonctionnels).

A ce jour, peu de travaux ont été menés pour trouver des composés alternatifs aux durcisseurs conventionnels, tout particulièrement des composés alternatifs biosourcés. Fache *et al.* se sont intéressés à la vanilline, dont nous reproduisons ci-après, à toutes fins utiles, la formule chimique : 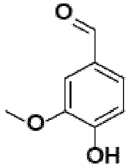 et ont proposé, dans European Polymer Journal, 2015, 73, 344-362, comme composé alternatif biosourcé aux durcisseurs conventionnels de résines époxy, la vanillylamine (synthétisée, *via* la vanillyloxime, à partir de ladite vanilline). Ladite vanillylamine, utilisée donc comme durcisseur de monomères ou prépolymères époxy thermodurcissables, ne permet pas l'obtention de polyépoxydes qui présentent de hautes propriétés mécaniques et de tenue en température.

Chapman et al. (J. Chem. Soc. (C) 1970, 6, 865-872) décrivent la synthèse d'acide indoline-2-carboxylique, de biphényles de bi-indolyles hydroxylés. Gaur et al. (Synthetic Metals 2010, 160, 2061-2064) rapportent les propriétés de dispositifs électroniques obtenus en utilisant du poly(3-hexylthiophène) comme agent réticulant. Ces deux documents décrivent le composé 5,5'-diamino-2,2',3,3'-tétraméthoxybiphényle. Maurer et al. (Journal Für Praktische Chemie 1935, 144(1-2), 41-48) décrivent des dérivés de divanilline, notamment le produit de réaction entre l'hydroxylamine et la divanilline méthylée. La polymérisation réticulante du diglycidyléther de bisphénol A (DGEBA) par un durcisseur conventionnel comme la diaminodiphénylsulfone (DDS) est illustrée par FR 2 734 832 A1.

Dans un tel contexte, il est du mérite des inventeurs de proposer des composés biphényle difonctionnels originaux (convenant comme durcisseurs (voir ci-dessus et ci-dessous)) et qui, pour nombre d'entre eux, peuvent être d'origine naturelle). Lesdits composés biphényle difonctionnels originaux répondent à la formule (I) ci-après : 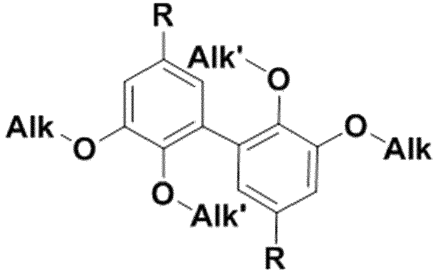 dans laquelle :
Alk est un groupe alkyle, linéaire ou ramifié, comportant de 1 à 6 atomes de carbone,
Alk' est un groupe alkyle, linéaire ou ramifié, comportant de 1 à 6 atomes de carbone, et
R est choisi parmi -CH₂-NH₂, -N=C=O et -NH₂.

La formule (I) ci-dessus confirme que les composés de l'invention sont des composés biphényle difonctionnels, difonctionnels de par la présence des deux groupes R (identiques). Lesdits deux groupes R sont choisis parmi les groupes aminométhyle (-CH₂-NH₂), isocyanate (-N=C=O) et amino (-NH₂). On comprend que ces groupes R conviennent pour réaction avec des monomères ou prépolymères thermodurcissables, notamment tels qu'identifiés ci-dessus.

R, groupe amino (directement porté par les noyaux phényle), est particulièrement préféré.

Pour ce qui concerne le groupe alcoxy, -O-Alk, tel que défini ci-dessus (Alk = groupe alkyle, linéaire ou ramifié, comportant de 1 à 6 atomes de carbone), il consiste avantageusement en un groupe alcoxy, -O-Alk_{inférieur}, ledit groupe alkyle inférieur ne comportant que 1 à 4 atomes de carbone, voire très avantageusement que 1 ou 2 atomes de carbone. On comprend plus loin que la valeur de Alk est fixée par la nature des produits de départ utilisés. Ainsi, à partir de l'éthyl vanilline de synthèse, on obtient des composés de formule (I) dans laquelle Alk=-C₂H₅ (plus généralement, à partir d'une (C₂-C₆)alkyl vanilline de synthèse, on obtient des composés de formule (I) dans laquelle Alk est un groupe alkyle en C₂-C₆), à partir de la vanilline (de synthèse ou, plutôt avantageusement d'origine naturelle), des composés de formule (I) dans laquelle Alk = -CH₃. Lesdits composés de formule (I) dans laquelle Alk = -CH₃ (Alk est un groupe méthyle) sont particulièrement préférés (notamment de par leur obtention possible à partir de vanilline naturelle et en référence à leurs viscosités). Ainsi la suite de la présente description et les exemples sont-ils largement développés, de façon nullement limitative, en référence à cette valeur préférée.

Pour ce qui concerne le groupe alcoxy, -O-Alk', tel que défini ci-dessus (Alk' = groupe alkyle, linéaire ou ramifié, comportant de 1 à 6 atomes de carbone), il consiste aussi avantageusement en un groupe alcoxy, -O-Alk_{inférieur}, ledit groupe alkyle inférieur ne comportant que 1 à 4 atomes de carbone, voire très avantageusement que 1 ou 2 atomes de carbone. On comprend plus loin que la valeur de Alk' est gérée par la nature exacte du iodoalcane utilisé pour alkyler (éthérifier les fonctions hydroxy en position 4,4' du dimère). Les composés de formule (I) dans laquelle Alk' = -CH₃ (Alk' est un groupe méthyle) sont particulièrement préférés (notamment en référence à leurs viscosités).

On a compris que Alk et Alk' sont indépendants (ce qui se confirme ci-après à la considération du procédé de préparation des composés de l'invention), qu'ils ne sont donc nullement obligatoirement identiques. Toutefois, dans le cadre d'une variante particulièrement préférée, on a Alk = Alk' = -CH₃ (= un groupe méthyle).

Ainsi, sont tout particulièrement préférés, les composés de formule (I) dans laquelle :
- Alk = Alk' = -CH₃ et R = -CH₂-NH₂, à savoir la 3,4-diméthoxydivanyllylamine (voir l'exemple 1 ci-après),
- Alk = Alk' = -CH₃ et R = -N=C=O, à savoir le 3,4-diméthoxydiphénylisocyanate (voir l'exemple 2 ci-après), et
- Alk = Alk' = -CH₃ et R = -NH₂, à savoir la 3,4-diméthoxydianiline (voir l'exemple 2 ci-après).

L'homme du métier a d'ores et déjà compris le grand intérêt des composés biphényle difonctionnels de l'invention, tout particulièrement comme durcisseurs de monomères ou prépolymères à polymériser (à réticuler) (*via* des fonctions adéquates, aptes à réagir, avec les groupes R réactifs desdits composés de l'invention). Par ailleurs, la présence des deux noyaux aromatiques dans la formule desdits composés s'est révélée particulièrement intéressante en référence aux propriétés mécaniques et à la tenue en température des produits, monomères ou prépolymères thermodurcissables thermodurcis (en utilisant lesdits durcisseurs), et la présence de la liaison Carbone-Carbone entre lesdits deux noyaux aromatiques s'est révélée étonnamment opportune en référence au taux de coke résiduel après dégradation thermique desdits produits. On se doit aussi d'insister sur l'obtention desdits composés biphényle difonctionnels de l'invention par des procédés de mise en œuvre non complexe et, possiblement, pour nombre desdits composés, à partir de la vanilline, produit naturel.

La préparation des composés de formule (I) constitue un autre objet de l'invention.

Le procédé de préparation en cause comprend avantageusement :
- la mise à disposition d'un produit choisi parmi la vanilline, les analogues de la vanilline présentant un groupe -O-(C₂-C₆)alkyle en position 3, les esters de la vanilline et les analogues desdits esters présentant un groupe -O-(C₂-C₆)alkyle en position 3 ;
- la dimérisation dudit produit pour l'obtention d'un dimère ; et
- le traitement dudit dimère obtenu pour la conversion de ses fonctions -OH phénoliques en fonctions alcoxy (-OAlk') et, soit de ses fonctions aldéhydes en fonctions aminométhyle (-CH₂-NH₂), soit de ses fonctions esters en fonctions isocyanate (-N=C=O) ou amino (-NH₂).

On se propose, à propos des réactifs et réactions en cause, de fournir, de façon non limitative, les informations ci-après.

La mise à disposition des produits de départ listés ci-dessus ne pose aucune difficulté particulière à l'homme du métier. Certains desdits produits sont des produits naturels (la vanilline ; ladite vanilline et ses esters directement obtenus à partir de celle-ci pouvant donc être qualifiée de biosourcés), certains sont commercialement disponibles, tous peuvent être préparés selon des voies de synthèse familières à l'homme du métier (voir ci-après).

Le procédé de l'invention est avantageusement mis en œuvre à partir de vanilline. Ladite vanilline est avantageusement d'origine naturelle, extraite de la biomasse (de la lignine, notamment du bois). Une telle vanilline biosourcée est commercialement disponible. Elle peut également être obtenue par synthèse, notamment à partir du phénol, *via* le guaiacol (chimie du phénol). Des procédés de synthèse de ce type sont, à ce jour, développés à l'échelle industrielle et par exemple décrits dans ACS Sustainabie Chem. Eng. 2016, 4, 35-46. De la vanilline synthétique est aussi commercialement disponible.

Pour ce qui concerne les analogues de la vanilline (présentant un groupe -O-(C₂-C₆)alkyle (= -O-alkyl, ledit groupe alkyle, linéaire ou ramifié, comportant de 2 à 6 atomes de carbone), avantageusement un groupe -Oéthyle, en position 3), ils peuvent être obtenus, sans difficultés particulières, dans le cadre de ces mêmes procédés de synthèse, en faisant intervenir, pour l'éthérification de la fonction OH concernée, en lieu et place du sulfate de diméthyle (Me₂SO₄) ou de l'alcool méthylique (MeOH), respectivement, du sulfate de di(C₂-C₆)alkyl ou de l'alcool (C₂-C₆)alkylique ((C₂-C₆)alkylOH). L'éthyl vanilline est par ailleurs commercialement disponible.

Le procédé de l'invention est également avantageusement mis en œuvre à partir d'un ester de vanilline (généralement un ester en C₁-C₆, avantageusement un ester en C₁-C₄, très avantageusement un ester en C₁-C₂, de préférence l'ester en C₁ (le vanillate de méthyle)). A la considération de la description détaillée du procédé de préparation des composés de l'invention qui suit, l'homme du métier concevra aisément le peu d'intérêt à utiliser un ester dont la fonction ester comporte plus d'un atome de carbone (la fonction ester étant destinée à être saponifiée). Un tel ester peut aisément être préparé, à partir de la vanilline (si celle-ci est biosourcée, ledit ester peut donc aussi être qualifié de biosourcé) selon des procédés décrits dans l'art antérieur, plus particulièrement un procédé en deux étapes qui comprend, successivement, une oxydation de la fonction aldéhyde de la vanilline pour l'obtention de l'acide vanillique puis une estérification dudit acide (en présence d'un alcool adéquat : (C₁-C₆)alkylOH, avantageusement CH₃OH (voir ci-dessus)) en milieu acide. Le vanillate de méthyle est, à ce jour, commercialement disponible.

Pour ce qui concerne les analogues des esters de vanilline (présentant un groupe -O-(C₂-C₆)alkyle (= -O-alkyl, ledit groupe alkyle, linéaire ou ramifié, comportant de 2 à 6 atomes de carbone), avantageusement un groupe -Oéthyle, en position 3), on comprend qu'ils sont obtenus par estérification des analogues correspondant de la vanilline (analogues obtenus par synthèse à partir du phénol (voir ci-dessus)).

Au vu des propos ci-dessus, on a compris que le produit de départ, utilisé pour la mise en œuvre du procédé de l'invention, est :
- avantageusement choisi par la vanilline et les esters de vanilline (généralement donc les esters en C₁-C₆, avantageusement en C₁-C₄, très avantageusement en C₁-C₂, de préférence l'ester en C₁ (le vanillate de méthyle)),
- très avantageusement choisi parmi la vanilline d'origine naturelle (biosourcée) et les esters obtenus à partir de la vanilline d'origine naturelle (biosourcée), esters pouvant donc être qualifiés de biosourcés.

La dimérisation des produits mis à disposition (produits de départ) ne pose pas de difficultés. Un couplage oxydatif est en cause. L'utilisation de la Laccase issue de *Trametes versicoior,* dans un tel contexte, a été largement décrite. Ainsi, les exemples 1 et 4 de la demande EP 3 002 333 illustrent la préparation, à partir, respectivement, de la vanilline et du vanillate de méthyle, de la divanilline et du divanillate de méthyle (exemple d'ester de divanilline).

Les dimères obtenus présentent des fonctions aldéhyde ou ester en positions 1 et 1' et des fonctions -OH phénoliques en positions 4 et 4'. Il convient alors de convertir, d'une part, lesdites fonctions -OH phénoliques en fonctions alcoxy -OAlk', et, d'autre part, lesdites fonctions aldéhyde ou ester en le groupe réactif attendu R. On convertit les fonctions aldéhyde en fonctions aminométhyle (-CH₂-NH₂) et les fonctions ester en fonctions isocyanate (-N=C=O) ou amino (-NH₂). On préfère généralement alkyler (éthérifier, de façon conventionnelle (voir ci-après)) les fonctions -OH phénoliques avant de convertir lesdites fonctions aldéhyde ou ester mais il est tout à fait possible d'alkyler lesdites fonctions-OH phénoliques au cours de la conversion desdites fonctions aldéhyde ou ester. Il n'est *a priori* pas exclu de mettre en œuvre l'alkylation après la conversion.

On propose ci-après de préciser, de façon nullement limitative, le procédé de l'invention, tout particulièrement sa dernière étape : étape de traitement du dimère qui inclut donc les deux types de conversion énoncées ci-dessus.

Selon une première variante, le produit mis à disposition est choisi parmi la vanilline et les analogues de la vanilline présentant un groupe -O-(C₂-C₆)alkyle en position 3. La dimérisation dudit produit conduit à un dimère choisi parmi la divanilline (présentant un groupe -O-CH₃ en positions 3 et 3') et les analogues de la divanilline (présentant un groupe -O-(C₂-C₆)alkyle en positions 3 et 3'). Pour ce qui concerne le traitement dudit dimère, il comprend :
- successivement, l'alkylation (l'éthérification) des fonctions -OH phénoliques dudit dimère puis la conversion des fonctions aldéhyde dudit dimère alkylé en fonctions oxime ou la conversion des fonctions aldéhyde dudit dimère en fonctions oxime puis l'alkylation (l'éthérification) des fonctions -OH phénoliques dudit dimère avec fonctions oxime, pour l'obtention d'une oxime divanillique alkylée ; avantageusement l'alkylation (l'éthérification) des fonctions -OH phénoliques dudit dimère puis la conversion des fonctions aldéhyde dudit dimère alkylé en fonctions oxime ; et
- la réduction de ladite oxime divanillique alkylée (obtenue) pour l'obtention d'une amine divanillique alkylée répondant à la formule (I) dans laquelle R = -CH₂-NH₂.

Ledit traitement dudit dimère comprend la préparation d'une oxime divanillique alkylée puis la réduction de celle-ci.

L'oxime divanillique alkylée est obtenue à l'issue des deux étapes indiquées : alkylation (éthérification) des fonctions -OH phénoliques + conversion des fonctions aldéhyde en fonctions oxime, mises en œuvre dans un ordre quelconque, de préférence dans celui indiqué comme avantageux. L'alkylation (on a vu qu'il s'agit plus exactement d'une éthérification : -OH devient -OAlk') ne soulève aucune difficulté particulière. Le dimère avec fonctions aldéhyde ou le dimère avec fonctions oxime (si la conversion a été mise en œuvre avant l'alkylation) est généralement mis en présence d'une base, telle le carbonate de potassium, dans un solvant (tel le diméthylformamide (DMF)) et un iodoalkyle (I-Alk', tel le iodométhane) est ajouté lentement. La réaction, à température élevée (par exemple 80°C) dure plusieurs heures. A l'issue de la réaction, on filtre avantageusement le milieu réactionnel et le composé alkylé (par le groupe Alk') est récupéré par précipitation dans l'eau froide. La conversion des fonctions aldéhyde du dimère ou du dimère alkylé (si l'alkylation a été mise en œuvre avant la conversion), de la même façon, ne soulève pas de difficultés particulières. Elle est généralement mise en œuvre en milieu légèrement basique (en présence d'acétate de sodium, par exemple), dans un solvant (éthanol, par exemple), en présence de chlorure d'hydroxylammonium. Elle est mise en œuvre à température élevée (par exemple 100°C), généralement pendant au moins une heure. Après extraction de la phase organique, on récupère l'oxime divanillique, alkylé ou non, renfermé dans celle-ci, par évaporation.

La réduction de l'oxime divanillique alkylée obtenue est généralement mise en œuvre sous pression d'hydrogène en présence d'un catalyseur d'hydrogénation, tel le nickel de Raney ou du palladium sur charbon actif.

A l'issue de cette réduction, on obtient un produit de l'invention, de formule (I) dans laquelle R = -CH₂-NH₂ ; les valeurs de Alk et Alk' dépendant, respectivement, de la nature exacte du produit de départ (vanilline, alors Alk = -CH₃, analogue de la vanilline, alors Alk = -(C₂-C₆)alkyl) et du iodoalkyle (I-Alk') utilisé en amont pour l'alkylation.

Selon une seconde variante, le produit mis à disposition est choisi parmi un ester (généralement en C₁-C₆, de préférence en C₁ (voir ci-dessus)) de vanilline (présentant donc un groupe -O-CH₃ en position 3, possiblement obtenu à partir de vanilline (avantageusement d'origine naturelle) ou commercialement disponible) et les analogues d'un tel ester (présentant donc une fonction -O-(C₂-C₆)alkyle en position 3). La dimérisation dudit produit conduit à un dimère choisi parmi le divanillate correspondant (présentant un groupe -O-CH₃ en position 3 et 3') et les analogues dudit divanillate correspondant (présentant un groupe -O-(C₂-C₆)alkyle en position 3 et 3'). Pour ce qui concerne le traitement dudit dimère, il comprend :
- successivement, la saponification dudit dimère pour obtenir un acide divanillique et l'alkylation (l'éthérification) des fonctions -OH phénoliques dudit acide ou l'alkylation (l'éthérification) des fonctions -OH phénoliques dudit dimère pour obtenir un ester divanillique alkylé et la saponification dudit ester divanillique alkylé, pour obtenir un acide divanillique alkylé ; avantageusement la saponification dudit dimère et l'alkylation (l'éthérification) des fonctions -OH de l'acide divanillique obtenu ;
- l'acylation dudit acide divanillique alkylé pour obtenir un diazoture d'acyle alkylé ; et
- la mise en œuvre d'un réarrangement de Curtius sur ledit diazoture d'acyle alkylé pour l'obtention d'un dialcoxydiphénylisocyanate répondant à la formule (I) dans laquelle R = -N=C=O ; et
- éventuellement, en sus, l'hydrolyse dudit dialcoxydiphényl isocyanate pour l'obtention d'une dianiline alkylée de formule (I) dans laquelle R = -NH₂.

Ledit traitement dudit dimère comprend la préparation d'un acide divanillique alkylé, l'acylation de celui-ci, sa conversion en un dialcoxydiphénylisocyanate répondant à la formule (I) dans laquelle R = -N=C=O et éventuellement la conversion dudit dialcoxydiphénylisocyanate répondant à la formule (I) dans laquelle R = -N=C=O en une dianiline alkylée de formule (I) dans laquelle R = -NH₂.

L'acide divanillique alkylé est obtenu à l'issue des deux étapes indiquées : saponification des fonctions ester + alkylation (éthérification) des fonctions -OH phénoliques, mises en œuvre dans un ordre quelconque, de préférence dans celui indiqué comme avantageux. La saponification est une réaction conventionnelle (elle est par exemple illustrée dans l'exemple 13 de la demande EP 3 002 333). Le dimère avec ses fonctions ester, éventuellement alkylé (si l'alkylation ou éthérification a été mise en œuvre avant la saponification) est généralement chauffé en milieu alcoolique (méthanol ou éthanol, par exemple) en présence d'une base forte, telle la soude ou la potasse. La réaction, à température élevée (généralement au reflux) dure plusieurs heures. L'alkylation ou éthérification est telle que décrite ci-dessus dans le contexte de la première variante.

L'acylation de l'acide divanillique alkylé obtenu est également mise en œuvre de façon conventionnelle. Ledit acide, dans un solvant (des mélanges THF + eau se sont révélés de très bons solvants), est généralement tout d'abord mis en présence d'une base (telle la triéthylamine) et d'un chlorure d'acyle (tel le chloroformiate d'éthyle). Ensuite, un azoture d'acyle, avantageusement l'azoture de sodium, est ajouté. Ladite réaction d'acylation est menée à froid (en deçà de la température ambiante (par exemple à 0°C), pour éviter tout risque d'emballement de celle-ci et minimiser la production de produits secondaires.

Le diazoture d'acyle alkylé obtenu doit alors être transformé en diisocyanate, *via* un réarrangement de Curtius. A cette fin, il est généralement, dissous dans un solvant sec (par exemple, du toluène distillé), sous atmosphère inerte (azote, par exemple), chauffé. Il est chauffé, avantageusement dans un tube de Schlenk, à une température élevée, par exemple de 80°C, pendant plusieurs heures. A l'issue de ce réarrangement de Curtius, on obtient un composé de l'invention, de formule (I) dans laquelle R = - N=C=O ; les valeurs de Alk et Alk' dépendant, respectivement, de la nature exacte du produit de départ (un ester de vanilline, alors Alk = -CH₃, un ester d'un analogue de vanilline, alors Alk = -(C₂-C₆)alkyl) et de celle du iodoalkyle (I-Alk') utilisé en amont pour l'alkylation.

Pour obtenir un composé de formule (I) dans laquelle R = -NH₂, on hydrolyse le diisocyanate obtenu à l'étape précédente (en présence d'eau). Selon une variante, le diisocyanate, en solution dans un solvant (par exemple, du toluène), est chauffé en présence d'une base (telle KOH) en solution aqueuse. Il est généralement chauffé à température élevée pendant plusieurs heures. On a compris que l'on obtient ledit composé de formule (I) dans laquelle R = -NH₂, avec les valeurs de Alk et Alk' fixées, respectivement, par la nature exacte du produit de départ et de celle du iodoalkyle utilisé en amont pour l'alkylation (voir ci-dessus).

A la considération de la description ci-dessus du procédé de l'invention, plus précisément de ses deux variantes de mise en œuvre, et des exemples 1 et 2 ci-après, on conçoit que des réactions de type connu *per se* sont agencées au sein de schémas réactionnels originaux pour conduire aux composés de l'invention.

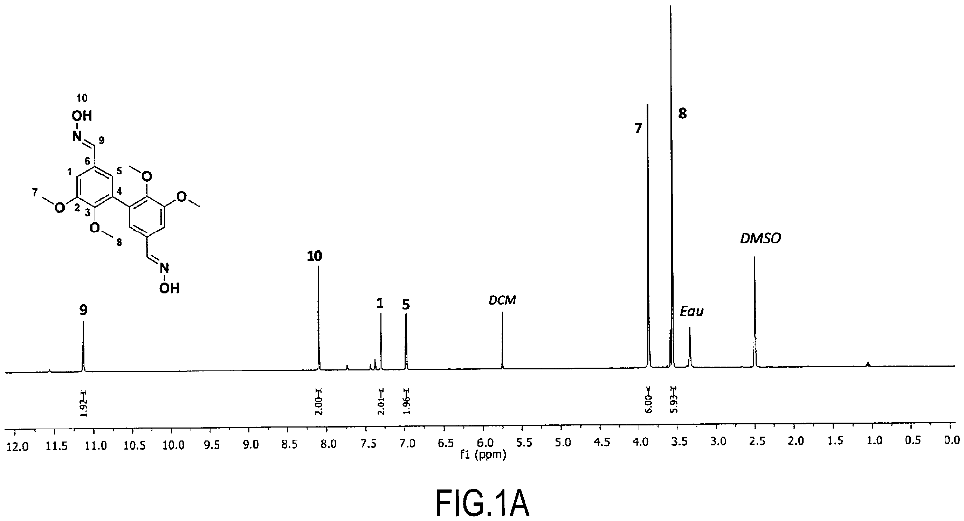

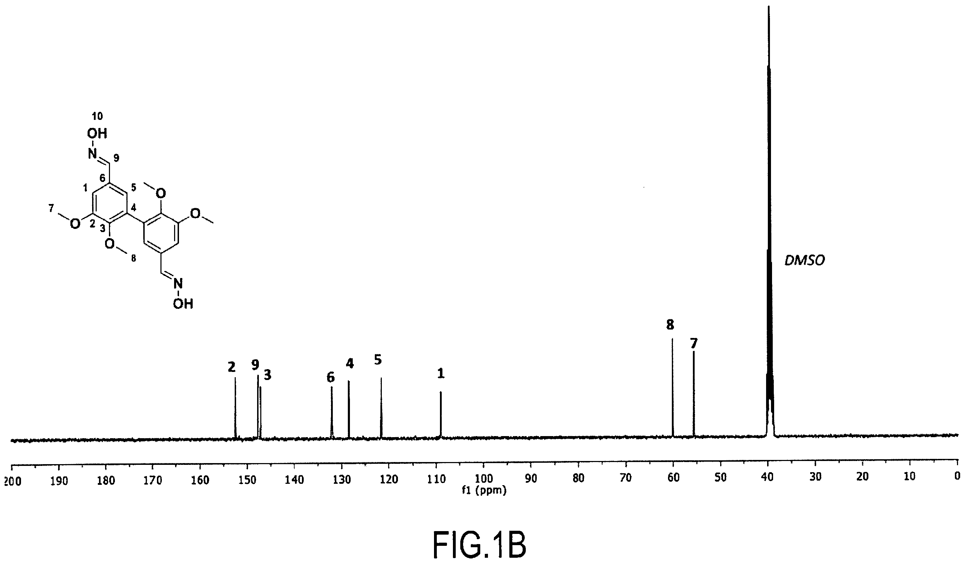

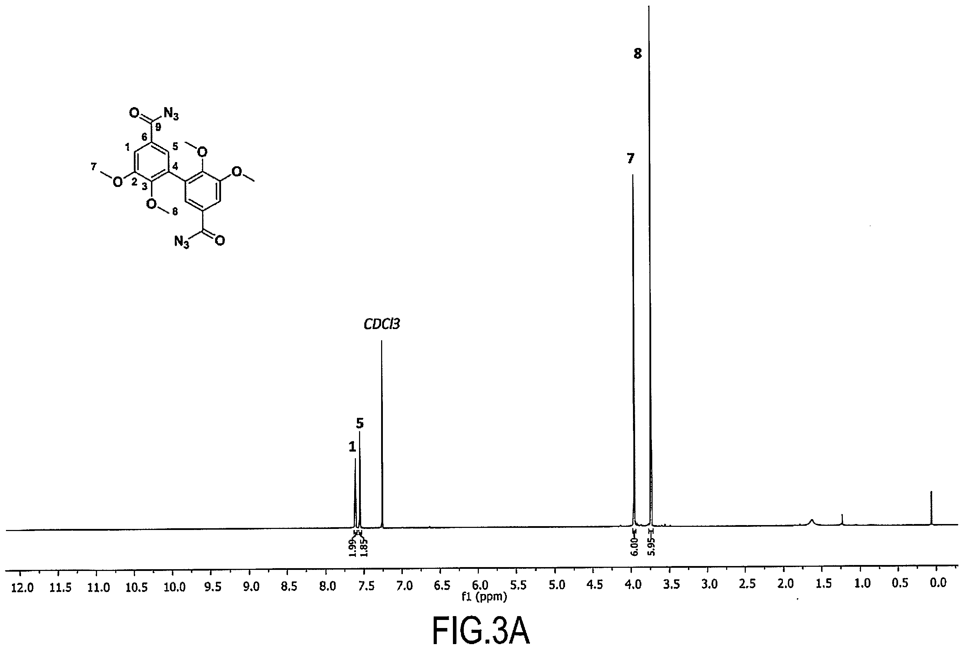

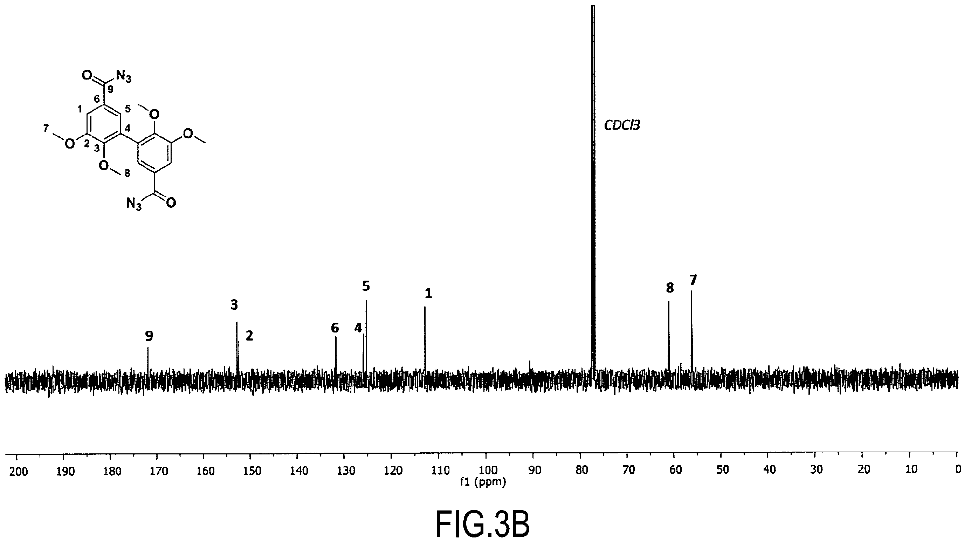

On propose ci-après les schémas réactionnels pour, à partir de la vanilline (avantageusement d'origine naturelle), obtenir les composés de formule (I) dans laquelle Alk = -CH₃ et Alk' = -CH₃. 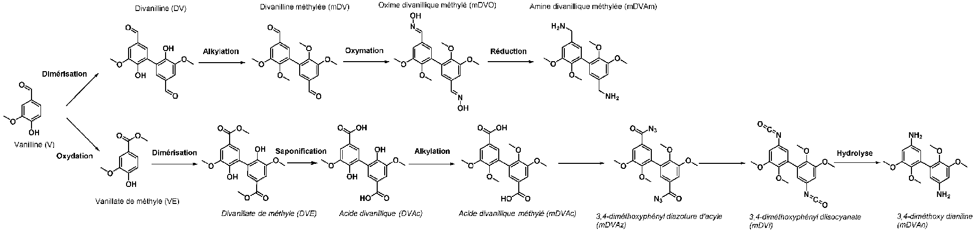 Selon un autre de ses objets, la présente invention concerne des produits intermédiaires, utiles à la préparation des composés de formule (I), produits intermédiaires avec fonctions oxime (fonctions -CH=N-OH, précurseur de R = CH₂NH₂) et produits intermédiaires avec fonctions azoture (fonctions -C(O)-N₃, précurseur de R = isocyanate (lui-même précurseur de R = -NH₂)). Elle concerne, en fait :
- les 3,4-di(C₁-C₆)alcoxydivanillique oxime (voir ci-dessus), à l'exception de la 3,4-diméthoxydivanillique oxime (voir ci-dessus, voir l'exemple 1c, voir les figures 1A et 1B), et
- les 3,4-di(C₁-C₆)alcoxydiphénylazoture d'acyle (voir ci-dessus), tout particulièrement le 3,4-diméthoxydiphénylazoture d'acyle (voir ci-dessus, voir l'exemple 2d, voir les figures 3A et 3B).

Selon un autre de ses objets, la présente invention concerne aussi les utilisations desdits composés de formule (I), décrits ci-dessus et/ou préparés selon le procédé décrit ci-dessus. Elle concerne tout particulièrement leur utilisation à titre de durcisseur (agent de polymérisation réticulante ou agent de réticulation difonctionnel) d'une résine thermodurcissable (à base de monomères ou prépolymères thermodurcissables (voir l'introduction du présent texte)). Ladite résine thermodurcissable peut notamment être choisie parmi :
- les résines époxy, les résines polycarbonate et les résines polyacide carboxylique pour l'obtention, respectivement, de polyépoxydes, de poly(hydroxy)uréthanes et de polyamides (résines thermodurcissables thermodurcies). On comprend bien évidemment que les durcisseurs de l'invention qui conviennent pour réagir avec les fonctions époxy, carbonate et acide carboxylique sont ceux de formule (I) dont le groupe R renferme ou consiste en une fonction amine (R= -CH₂-NH₂ et R = -NH₂) ;
- les résines polyol et les résines polyamine, pour l'obtention, respectivement, de polyuréthanes et de polyamides. On comprend bien évidemment que les durcisseurs de l'invention qui conviennent pour réagir avec les fonctions hydroxy et les fonctions amine sont ceux de formule (I) dont le groupe R est isocyanate.

Selon son dernier objet, l'invention concerne lesdites résines thermodurcissables thermodurcies en utilisant un composé de formule (I), tel que décrit ci-dessus et/ou préparé selon le procédé décrit ci-dessus, comme durcisseur. Elle concerne les résines thermodurcies, obtenues par traitement thermique, en présence d'au moins un durcisseur choisi parmi les composés de formule (I), tels que décrits ci-dessus et/ou préparés selon le procédé décrit ci-dessus, d'une résine thermodurcissable, notamment choisie parmi les résines époxy, les résines polycarbonate, les résines polyacide carboxylique (ladite résine thermodurcie étant alors, respectivement, de type polyépoxyde, poly(hydroxy)uréthane ou polyamide), les résines polyol et les résines polyamine (ladite résine thermodurcie étant alors, respectivement, de type polyol ou polyamide). On comprend bien évidemment que la résine thermodurcissable précurseur a été thermodurcie en utilisant le durcisseur de l'invention adéquat (voir ci-dessus). On comprend que le squelette de ladite résine thermodurcie comprend des motifs correspondant à la résine thermodurcissable « précurseur » (dont les fonctions réactives (époxy, carbonate, acide carboxylique, alcool, amine...) ont réagi avec les fonctions réactives R du durcisseur) et des motifs correspondant au durcisseur (dont les fonctions réactives R ont réagi avec les fonctions réactives de la résine thermodurcissable « précurseur » (époxy, carbonate, acide carboxylique, alcool, amine...).

Parmi ces résines (thermodurcissables) thermodurcies, sont particulièrement préférées celles de type polyépoxyde, obtenues par traitement thermique d'une résine époxy thermodurcissable (par exemple de type DGEBA) en utilisant un composé de formule (I) dans laquelle R = -CH₂-NH₂ ou -NH₂ (avantageusement un composé de formule (I) dans laquelle R = -NH₂, très avantageusement la 3,4-diméthoxydianiline), tel que décrit ci-dessus et/ou préparé selon le procédé décrit ci-dessus, comme durcisseur. Sont tout particulièrement préférées celles de type polyépoxyde obtenues par traitement thermique d'une résine époxy thermodurcissable renfermant au moins un composé biphényle pluriépoxydé choisi parmi :
- le diglycidyl éther de bisphénol, monomère (DGEBA) ou oligomère,
- le diglycidyl éther d'alcool divanillique (DiGEDVA),
- le triglycidyl éther d'alcool divanillique (TriGEDVA),
- le tétraglycidyl éther d'alcool divanillique (TetraGEDVA), et
- les mélanges d'au moins deux desdits glycidyl éthers d'alcool divanillique.

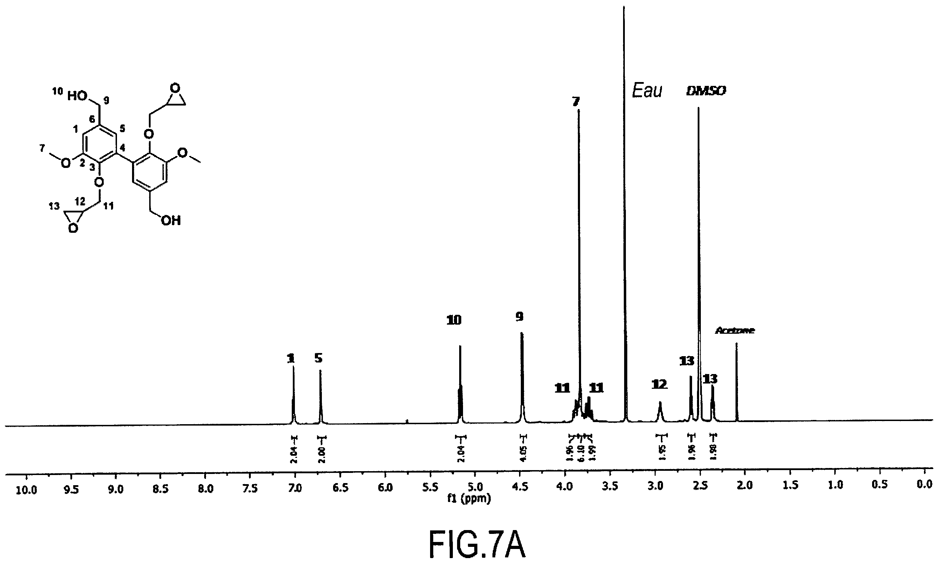

On a vu que le monomère ou prépolymère de type DGEBA était, à ce jour, très utilisé. La Demanderesse a décrit les di-, tri- et tétraglycidyl éthers d'alcool divanillique (et leurs mélanges) ainsi que d'autres composés biphényle pluriépoxydés dans la demande de brevet FR 17 60451 non publiée à ce jour. Tous les composés biphényle pluriépoxydés décrits, et tout particulièrement lesdits di-, tri- et tétraglycidyl éthers d'alcool divanillique (en mélange ou pris isolément), constituent des monomères ou prépolymères, de type résine époxy, thermodurcissables intéressants. Pour ce qui concerne lesdits di-, tri- et tétraglycidyl éthers d'alcool divanillique (et leurs mélanges), ils sont particulièrement intéressants, d'où présentement leur association vivement préconisée avec les durcisseurs de l'invention. Les formules chimiques desdits glycidyl éthers sont montrées dans le schéma réactionnel ci-après ainsi que dans la partie exemple ci-après ; les spectres ¹H et ¹³C RMN desdits glycidyl éthers sont donnés dans lesdits exemples et montrés sur les figures 7A-7B, 8A-8B et 9A-9B. Un de leur procédé de préparation, dont le schéma réactionnel est proposé ci-après : 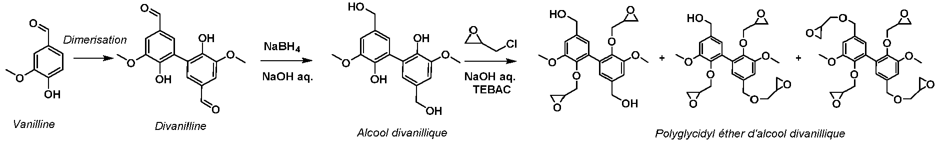 est précisément décrit dans ladite partie exemple ci-après (exemple 3).

On conçoit aisément qu'en faisant réagir, en lieu et place de l'épichlorhydrine (de formule Cl-CH₂-époxy), un analogue de celle-ci (de formule Cl-[CH_{2]n}-époxy, n entier de 0 à 6), on obtient d'autres éthers d'alcool divanillique, susceptibles de constituer des composés biphényle pluriépoxydés pouvant, aussi, être thermodurcis (réticulés) avec les durcisseurs de l'invention.

On peut également incidemment noter que les fonctions alcool -CH₂OH et hydroxy -OH de l'alcool divanillique peuvent aussi être éthérifiées en deux étapes (allylation + époxydation).

A la considération de ce qui précède, on comprend que des polyépoxydes peuvent ainsi être obtenus avec des résines époxy biosourcées et des durcisseurs biosourcés, résines et durcisseurs (biosourcés) étant obtenus à partir de vanilline.

L'invention est maintenant illustrée par les exemples ci-après et les figures annexées.
Les figures 1A et 1B montrent des spectres RMN ¹H et ¹³C de la 3,4-diméthoxydivanillique oxime (intermédiaire).
Les figures 2A et 2B montrent des spectres RMN ¹H et ¹³C de la 3,4-diméthoxydivanyllylamine (composé de l'invention (exemple 1)).
Les figures 3A et 3B montrent des spectres RMN ¹H et ¹³C du 3,4-diméthoxydiphénylazoture d'acyle (intermédiaire).
Les figures 4A et 4B montrent les spectres RMN ¹H et ¹³C du 3,4-diméthoxydiphénylisocyanate (composé de l'invention (exemple 2)).
Les figures 5A et 5B montrent les spectres RMN ¹H et ¹³C de la 3,4-diméthoxydianiline (composé de l'invention (exemple 2)).
La figure 6 montre le résultat d'une chromatographie menée sur un mélange de composés pluriépoxydés (de polyglycidyl éthers d'alcool divanillique) (exemple 3).
Les figures 7A à 9A sont des spectres RMN ¹H desdits composés pluriépoxydés isolés;
les figures 7B à 9B sont des spectres RMN ¹³C desdits composés pluriépoxydés.

### Exemple 1

### Synthèse de la [3-[5-(aminométhyl)-2,3-diméthoxy-phényl]-4,5-diméthoxy-phényl]méthanamine (3,4-diméthoxydivanillylamine) (de formule (I) dans laquelle Alk = Alk' = -CH₃ et R = -CH₂-NH₂) à partir de la divanilline (DV)

Les différentes étapes du schéma réactionnel ci-dessous (correspondant à celui de la première ligne de la page 15) ont successivement été mises en œuvre.

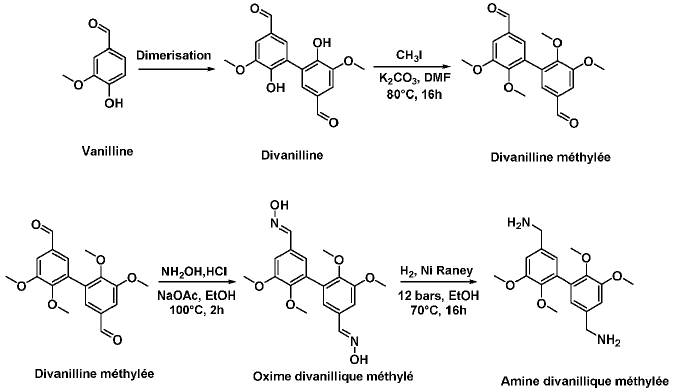

### la. Synthèse de la divanilline (DV)

Pour la préparation de la divanilline, on a procédé selon le mode opératoire décrit dans l'exemple 1 de la demande de brevet EP 3 002 333. Plus précisément, on a procédé comme suit.

La vanilline (20 g) (celle utilisée, commercialisée par la société Acros, n'était pas biosourcée. A toutes fins utiles, on indique qu'aurait pu être utilisée celle, biosourcée, commercialisée par la société Borregaard (NO)) a été solubilisée dans de l'acétone (160 mL) et une solution tampon d'acétate (1,5 L, préparée à partir de 2,63 g d'acide acétique et 8,4 g d'acétate de sodium). Au mélange résultant, a été ajoutée la laccase issue de *Trametes Versicolor* (170 mg). Pour être recyclée sous forme active, ladite laccase a besoin d'oxygène. Le milieu réactionnel a donc été laissé sous agitation avec un bullage d'air constant pendant 24 heures. La divanilline a ensuite été récupérée par filtration de la solution tampon sur filtre Büchner. Le filtrat a été récupéré et réutilisé pour d'autres réactions de dimérisation.

### 1a'. Purification de la divanilline (DV) synthétisée

Des traces de vanilline étaient susceptibles d'être présentes dans la divanilline récupérée. Pour les éliminer, ladite divanilline a été solubilisée dans une solution aqueuse de NaOH (200 mL à 0,5 M ; quelques gouttes de solution 5M ont été opportunément ajoutées pour faciliter la solubilisation). Un large volume d'éthanol (600 mL) a ensuite été ajouté à la solution ainsi qu'une solution aqueuse d'acide chlorhydrique (115 mL à 2 M) jusqu'à atteindre, pour le mélange, un pH = 3. En effet, la divanilline et la vanilline sont toutes les deux solubles à pH basique dans l'éthanol. En revanche, la divanilline n'est pas soluble dans l'éthanol à pH acide, contrairement à la vanilline. L'ajout de l'acide permet donc de séparer les deux produits par précipitation de la divanilline.

Le produit obtenu a été filtré, puis séché à l'étuve, afin d'enlever toute trace de solvant.

Les opérations de synthèse et purification ont été répétées. Le rendement était à chaque fois d'environ 95 %.

On a confirmé l'obtention de la divanilline (DV) par spectroscopie RMN :

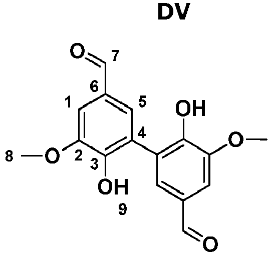

**RMN ¹H** (400 MHz, DMSO-d6, δ (ppm) : δ 9.69 (s, H₇), 7.57 (d, H₁), 7.16 (d, H₅), 3.76 (s, H₈).

**RMN ¹³C** (400 MHz, DMSO-d6, δ (ppm)) : δ 191.62 (s, C₇), 150.88 (s, C₃), 148.61 (s, C₂), 128.64 (s, C₆), 128.21 (s, C₄), 125.02 (s, C₅), 109.6 (s, C₁), 56.25 (C₈).

### 1b. Synthèse du 3-(5-formyl-2,3-diméthoxy-phényl)-4,5-diméthoxy-benzaldéhyde (divanilline méthylée) (mDV)

On a procédé selon le mode opératoire décrit dans l'exemple 9 de la demande de brevet EP 3 002 333. Plus précisément, on a procédé comme suit. 26 mmol de divanilline (≈ 8 g) et 15,2 g de carbonate de potassium (110 mmol) ont été dissous dans 120 mL de DMF. 9,6 ml d'iodométhane (158 mmol) ont ensuite été lentement ajoutés au mélange. Après 16 h d'agitation à 80°C, le mélange a été filtré et la solution résultante a été versée dans de l'eau froide. Le composé méthylé a précipité et a été récupéré par filtration et séché sous vide. Rendement de 80 %.

On a confirmé l'obtention de la divanilline méthylée (mDV) par spectroscopie RMN :

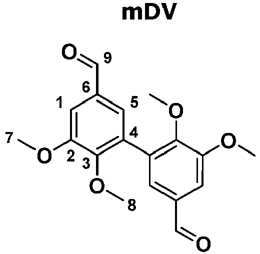

**RMN ¹H** (400 MHz, DMSO-d6, δ (ppm)) : δ 9.94 (d, H₉), 7.58 (d, H₁), 7.45 (d, H₅), 3.95 (s, H₇), 3.67 (s, H₈).

**RMN** ¹³C (400 MHz, DMSO-d6, δ (ppm)) : δ 152.00 (s, C₃), 144.81 (s, C₂), 137.64 (s, C₆), 132.12 (s, C₄), 120.33 (s, C₅), 110.18 (s, C₁), 62.63 (s, C₉), 59.91 (s, C₈), 55.52 (C₇).

### 1c. Synthèse de la (1E)-3-[5-[(E)-hydroxyiminométhyl]-2,3-diméthoxy-phényl]-4,5-diméthoxy-benzaldéhyde oxime (oxime divanillique méthylée) (mDVO)

1 g de chlorure d'hydroxylammonium (7 mmol) et 2 g d'acétate de sodium (12 mmol) ont été dissous dans 20 mL d'éthanol (+ 4 mL d'eau). 2 g de divanilline méthylée (6 mmol) ont ensuite été ajoutés au mélange. Après 2 h à 100°C, le mélange a été extrait au dichlorométhane (DCM) et lavé à l'eau. La phase organique a été évaporée en utilisant un évaporateur rotatif. Le produit récupéré a ensuite été séché sous vide. Rendement de 85 %. On a confirmé l'obtention de l'oxime de divanilline méthylée (mDVO) par spectroscopie RMN :

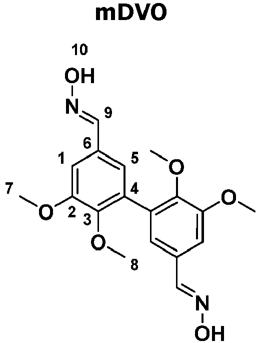

**RMN ¹H** (400 MHz, DMSO-d6, δ (ppm)) : δ 11.58 (s, H₁₀), 8.10 (s, H₉), 7.30 (d, H₁), 6.98 (d, H₅), 3.87 (s, H₇), 3.56 (s, H₈).

**RMN** ¹³C (400 MHz, DMSO-d6, δ (ppm)) : δ 152.66 (s, C₂), 147.81 (s, C₉), 147.25 (s, C₃), 131.87 (s, C₆), 128.69 (s, C₄), 121.69 (s, C₅), 108.78 (s, C₁), 59.88 (s, C₈), 55.6 (s, C₇).

Les spectres sont respectivement montrés sur les figures 1A et 1B.

### 1d. Synthèse de la [3-[5-(aminométhyl)-2,3-diméthoxy-phényl]-4,5-diméthoxy-phényl]méthanamine (amine divanillique méthylée) (mDVAm)

1 g d'oxime divanillinique méthylée (2,7 mmol) et 1 mL de nickel de Raney ont été solubilisés dans 30 mL d'éthanol. Le mélange a été placé dans un réacteur pressurisé sous 12 bars d'hydrogène. Après 16 h à 70°C, le mélange a été filtré et l'éthanol a été évaporé sous vide. Le produit résultant a été solubilisé dans le dichlorométhane (DCM) et lavé à l'eau. Le DCM a ensuite été évaporée sous vide. Rendement de 70 %.
On a confirmé l'obtention de l'amine divanillique méthylée (mDVAm) par spectroscopie RMN :

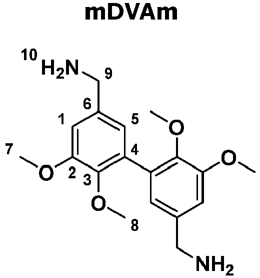

**RMN ¹H** (400 MHz, DMSO-d6, δ (ppm)) : δ 7.04 (m, H₅), 6.69 (m, H₁), 3.79 (m, H₈), 3.63 (s, H₇), 3.48 (m, H₉).

**RMN ¹³C** (400 MHz, DMSO-d6, δ (ppm)) : δ 151.75 (s, C₂), 144.46 (s, C₃), 136.04 (s, C₆), 132.36 (s, C₄), 121.78 (s, C₅), 111.41 (s, C₁), 60.05 (s, C₈), 55.65 (s, C₇), 51.62 (s, C₉).

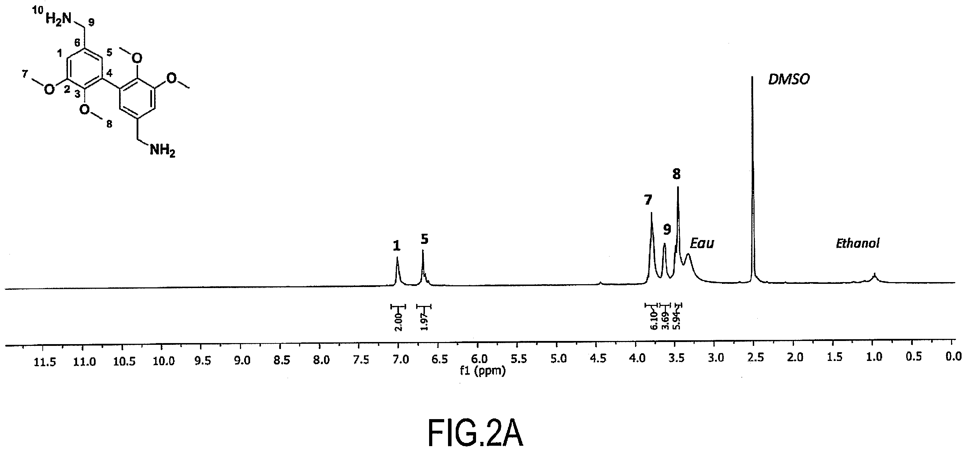

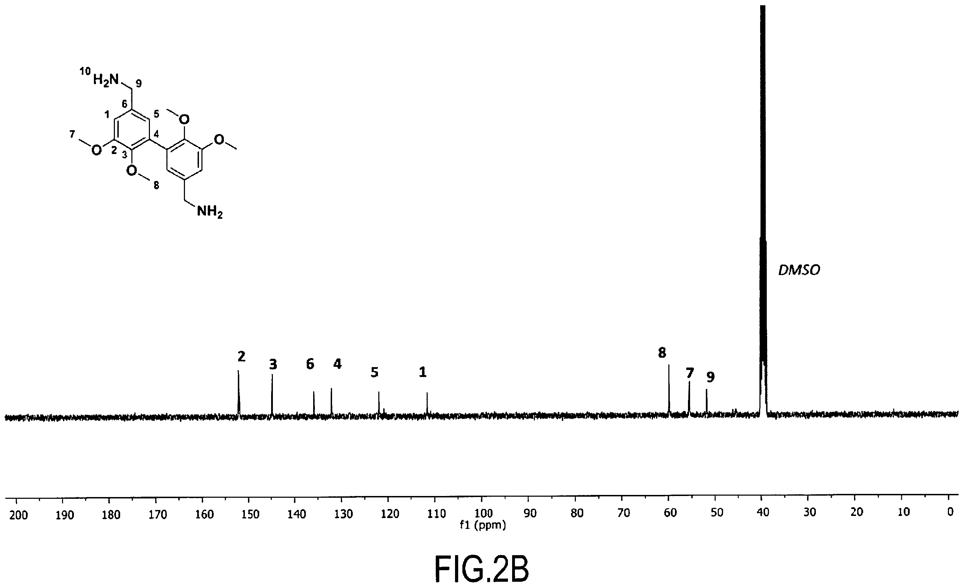

Les spectres sont respectivement montrés sur les figures 2A et 2B.

### Exemple 2

### Synthèse du 5-isocyanato-1-(5-isocyanato-2,3-diméthoxy-phényl)-2,3-diméthoxy-benzène (3,4-diméthoxydiphénylisocyanate) et de la 3-(5-amino-2,3-diméthoxy-phényl)-4,5-diméthoxy-aniline (3,4-diméthoxydianiline) (de formule (I) dans laquelle, respectivement, Alk = Alk' = -CH₃ et R = -N=C=O et Alk = Alk' = -CH₃ et R = -NH₂) à partir du vanillate de méthyle

Les différentes étapes du schéma réactionnel ci-dessous (correspondant à celui de la seconde ligne de la page 15) ont successivement été mises en œuvre.

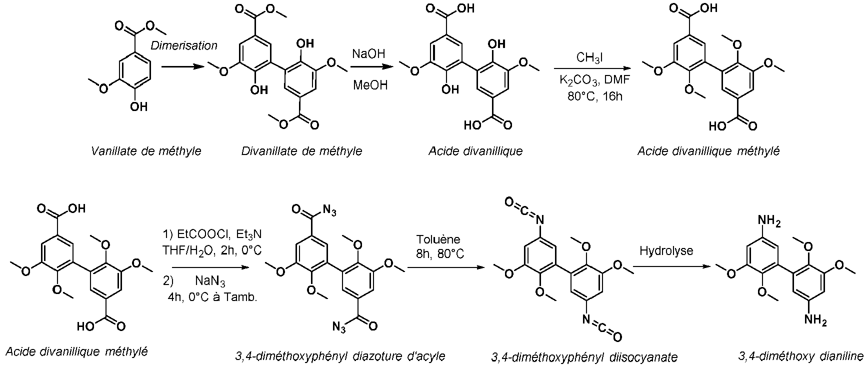

**2a. Synthèse du 4-hydroxy-3-(2-hydroxy-3-méthoxy-5-méthoxycarbonyl-phényl)-5-méthoxy-benzoate de méthyle (divanillate de méthyle) (DVE)** Pour la préparation du divanillate de méthyle, à partir du vanillate de méthyle (VE) (distribué par la société Sigma-Aldrich), on a procédé selon un mode opératoire tout à fait similaire à celui décrit pour la préparation de la divanilline au point 1.a ci-dessus.

On a procédé en fait (pour cette dimérisation) selon le mode opératoire décrit dans l'exemple 4 de la demande de brevet EP 3 002 333.

On a confirmé l'obtention du divanillate de méthyle par spectroscopie RMN :

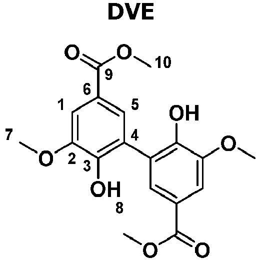

**RMN ¹H** (400 MHz, DMSO-d6, δ (ppm)) : δ 9.51 (s, H₈), 7.46 (d, H₁), 7.45 (d, H₅), 3.90 (s, H₇), 3.80 (s,H₁₀).

**RMN** ¹³C (400 MHz, DMSO-d6, δ (ppm)) : δ 166.09 (s, C₉), 148.88 (s, C₃), 147.47 (s, C₂), 125.40 (s, C₅), 124.36 (s, C₆), 119.48 (s, C₄), 110.92 (s, C₁), 56.01 (s, C₇), 51.79 (s,C₁₀).

### 2b. Synthèse de l'acide 3-(5-carboxy-2-hydroxy-3-méthoxy-phényl)-4-hydroxy-5-méthoxy-benzoïque (acide divanillique) (DVAc)

On a procédé, pour cette saponification, selon le mode opératoire décrit dans l'exemple 13 de la demande de brevet EP 3 002 333. Plus précisément, on a procédé comme suit.

10 mmol de divanillate de méthyle (≈ 2,5 g) ont été dissous dans 30 mL de méthanol. 3 g de soude (75 mmol) ont été ajoutés à la solution. La solution résultante a été chauffée au reflux pendant 4 h. On a stoppé la réaction en ajoutant au milieu réactionnel 2,5 mL d'eau. La phase aqueuse a été acidifiée avec de l'acide chlorhydrique et le diacide généré a précipité. Rendement de 92 %.

On a confirmé l'obtention de l'acide divanillique (DVAc) par spectroscopie RMN :

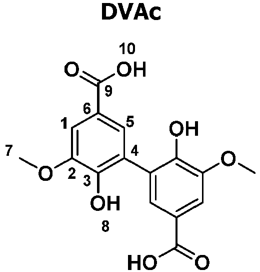

**RMN ¹H** (400 MHz, DMSO-d6, δ (ppm) : δ 9.39 (s, H₈), 7.45 (d, H₁), 7.41 (d, H₅), 3.89 (s, H₇).

**RMN ¹³C** (400 MHz, DMSO-d6, δ (ppm)) : δ 167.18 (s, C₉), 148.36 (s, C₃), 147.22 (s, C₂), 125.44 (s, C₆), 124.19 (s, C₄), 120.44 (s, C₅), 111.05 (s, C₁), 55.89 (s, C₇).

### 2c. Synthèse de l'acide 3-(5-carboxy-2,3-diméthoxy-phényl)-4,5-diméthoxy-benzoïque (acide divanillique méthylé) (mDVAc)

On a procédé, pour l'éthérification, comme décrit ci-dessus au point 1b, i.e. selon le mode opératoire décrit dans l'exemple 9 de la demande de brevet EP 3 002 333. Plus précisément, on a procédé comme suit.

26 mmol d'acide divanillique et 15,2 g de carbonate de potassium (110 mmol) ont été dissous dans 120 mL de DMF. 9,6 ml d'iodométhane (158 mmol) ont ensuite été lentement ajoutés au mélange. Après 16 h à 80°C, le mélange a été filtré et la solution résultante a été versée dans de l'eau froide. Le composé méthylé a précipité et a été récupéré par filtration et séché sous vide. Le rendement typique était de 80 %.
On a confirmé l'obtention de l'acide divanillique méthylé (mDVAc) par spectroscopie RMN :

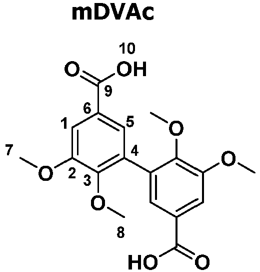

**RMN ¹H** (400 MHz, DMSO-d6, δ (ppm)): δ 12.94 (s, H₁₀), 7.58 (d, H₁), 7.39 (d, H₅), 3.91 (s, H₇), 3.61 (s, H₈).

**RMN ¹³C** (400 MHz, DMSO-d6, δ (ppm)): δ 166.83 (s, C₉), 152.20 (s, C₃), 150.07 (s, C₂), 131.34 (s, C₆), 125.91 (s, C₄), 124.13 (s, C₅), 112.93 (s, C₁), 60.28 (s, C₈), 55.87 (s, C₇).

### 2d. Synthèse de l'azoture de 3-(5-carbonazidoyl-2,3-diméthoxy-phényl)-4,5-diméthoxy-benzoyle (3,4-diméthoxydiphénylazoture d'acyle) (mDVAz)

3 mmol d'acide divanillique méthylé ont été dissous dans 15 mL de THF et 5 mL d'eau. La solution a été refroidie à 0 °C et 2,4 ml de triéthylamine dans 4 mL de THF ont été ajoutés goutte à goutte au mélange. 1,8 mL de chloroformiate d'éthyle ont ensuite été ajoutés au mélange. Le mélange résultant a alors été agité pendant 2 h à 0°C. Une solution d'azoture de sodium (1,2 g dans 4 mL d'eau) a été ajoutée goutte à goutte audit mélange et agitée pendant 2 h à 0°C, puis laissé 2 h à température ambiante. De l'eau froide a ensuite été progressivement ajoutée au milieu réactionnel pour faire précipiter le solide. Le précipité a été filtré, puis dissout dans le diclorométhane (DCM), lavé à l'eau. La phase organique a été évaporée sous évaporateur rotatif. Rendement de 60 %.

On a confirmé l'obtention du 3,4-diméthoxyphényl diazoture d'acyle (mDVAz) par spectroscopie RMN :

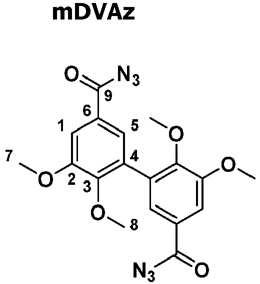

**RMN ¹H** (400 MHz, CDCl3, δ (ppm)): δ 7.61 (d, H₁), 7.55 (d, H₅), 3.96 (s, H₇), 3.74 (s, H₈).

**RMN ¹³C** (400 MHz, CDCl3, δ (ppm)): δ 171.85 (s, C₉), 152.79 (s, C₃), 152.39 (s, C₂), 131.69 (s, C₆), 125.81 (s, C₄), 125.23 (s, C₅), 112.78 (s, C₁), 61.10 (s, C₈), 56.22 (s, C₇).

Les spectres sont respectivement montrés sur les figures 3A et 3B.

### 2e. Synthèse du 5-isocyanato-1-(5-isocyanato-2,3-dimethoxy-phenyl)-2,3-dimethoxy-benzene (3,4-diméthoxydiphénylisocyanate) (mDVI)

Dans un tube de Schlenk sous atmosphère inerte (azote), 0,5 mmol de 3,4-diméthoxyphényl diazoture d'acyle ont été dissous dans 3 mL de toluène sec. Le mélange a été agité et chauffé à 80°C pendant 8 h. Le toluène a ensuite été évaporé sous évaporateur rotatif à 60°C. Rendement de 80 %.

On a confirmé l'obtention du 3,4-diméthoxydiphénylisocyanate (mDVI) par spectroscopie RMN :

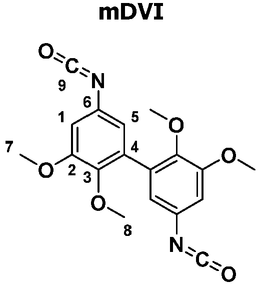

**RMN ¹H** (400 MHz, CDCl3, δ (ppm)): δ 6.65 (d, H₁), 6.58 (d, H₅), 3.88 (s, H₇), 3.64 (s, H₈).

**RMN ¹³C** (400 MHz, CDCl3, δ (ppm)): δ 153.41 (s, C₃), 144.82 (s, C₂), 132.46 (s, C₆), 128.72 (s, C₄), 124.71 (s, C₉), 118.91 (s, C₅), 108.86 (s, C₁), 60.99 (s, C₈), 56.13 (s, C₇).

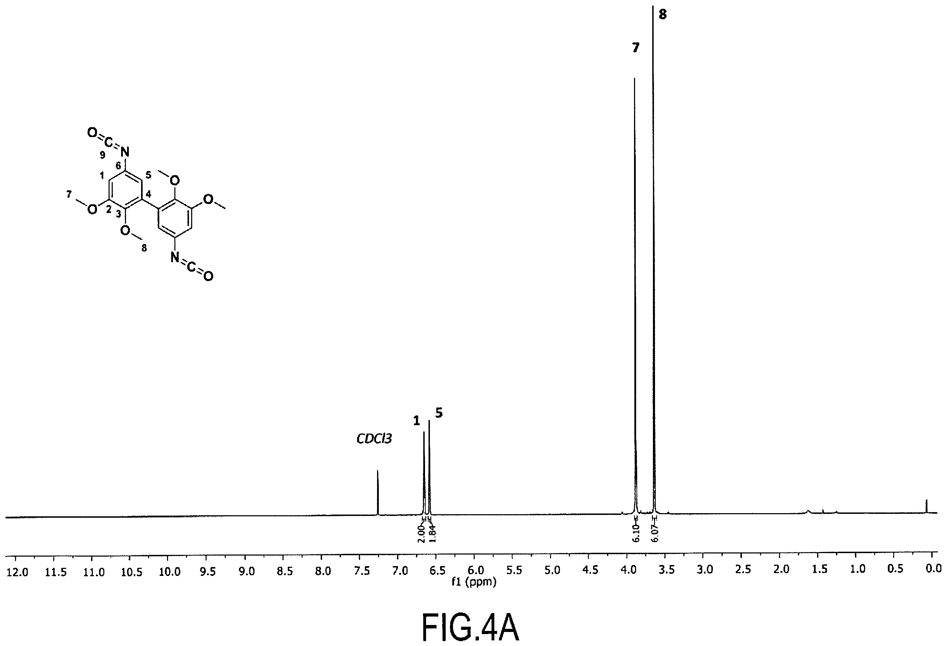

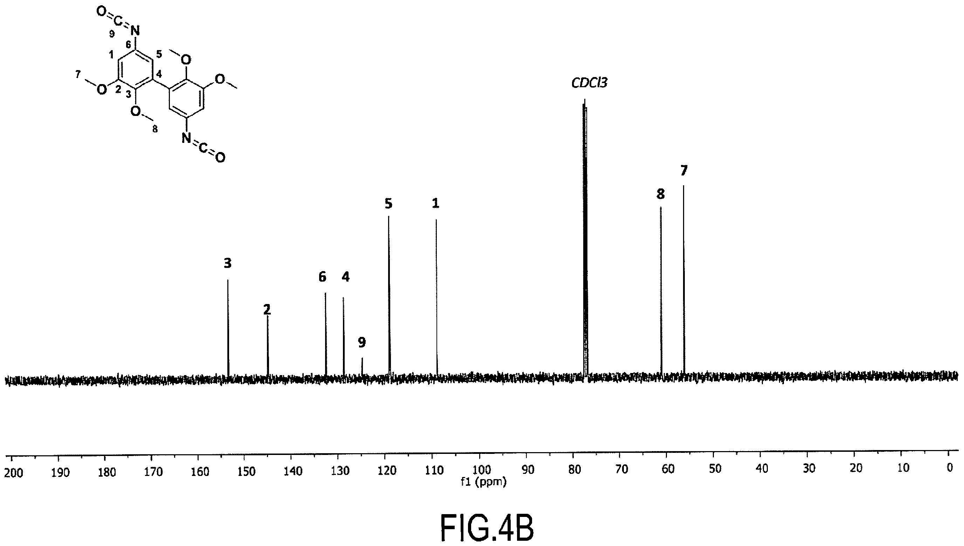

Les spectres sont respectivement montrés sur les figures 4A et 4B.

### 2f. Synthèse de la 3-(5-amino-2,3-dimethoxy-phenyl)-4,5-dimethoxy-aniline (3,4-diméthoxydianiline) (mDVAn)

3 mmol d'hydroxyde de potassium ont été ajoutés à 0,75 mmol de 3,4-diméthoxyphényl diisocyanate en solution dans le toluène. Le mélange a été agité et chauffé 12 h à 80°C. Le toluène a été évaporé sous vide. Le produit résultant a été solubilisé dans l'acétate d'éthyle et lavé à l'eau. Puis, la phase organique a été évaporée sous évaporateur rotatif. Rendement de 10 %.

On a confirmé l'obtention de la 3,4-diméthoxyphényldianiline (mDVAn) par spectroscopie RMN :

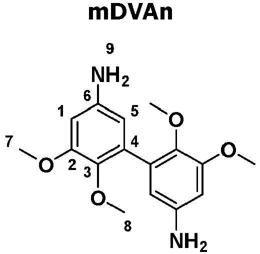

**RMN ¹H** (400 MHz, CDCl3, δ (ppm)): δ 6.23 (d, H₁), 5.90 (d, H₅), 4.79 (s, H₉), 3.72 (s, H₇), 3.38 (s, H₈).

**RMN ¹³C** (400 MHz, CDCl3, δ (ppm)): δ 152.47 (s, C₃), 144.22 (s, C₂), 136.90 (s, C₆), 133.35 (s, C₄), 107.48 (s, C₅), 98.42 (s, C₁), 59.97 (s, C₈), 55.18 (s, C₇).

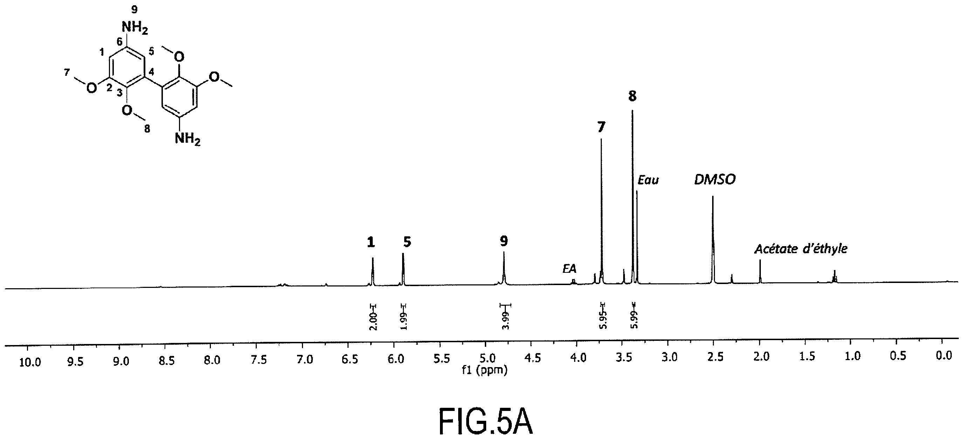

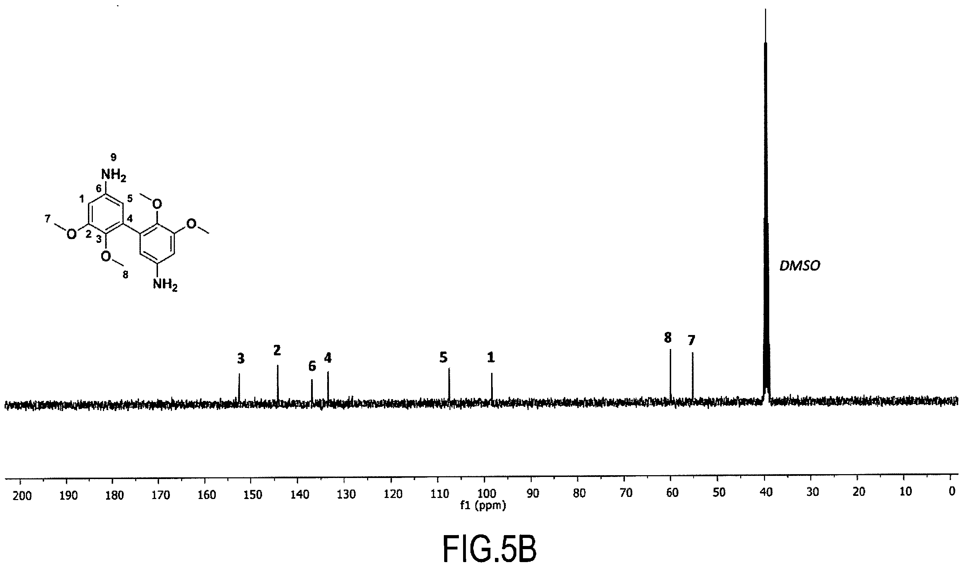

Les spectres sont respectivement montrés sur les figures 5A et 5B.

### Exemple 3

### Polyépoxydes obtenus à partir d'une résine époxy thermodurcissable et d'un durcisseur

- Les résines époxy utilisées ont été :
   - le diglycidyl éther de bisphénol-A (DGEBA), de formule : 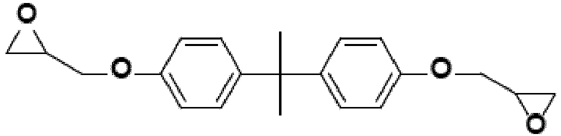 commercialisé par Sigma-Aldrich sous la dénomination commerciale D.E.R.^{®} 332, et
   - le tétraglycidyléther de l'alcool divanillique (TétraGEDVA), dont la préparation et la formule sont précisées ci-après.

Le TétraGEDVA a été obtenu (tout comme ces homologues : di- et triépoxydés (DiGEDVA et TriGEDVA) (voir ci-après)) dans les conditions précisées ci-dessous, à partir de divanilline, synthétisée et purifiée dans les conditions décrites à l'exemple 1 ci-dessus (plus précisément aux points la et 1a'dudit exemple 1). A) A partir de ladite divanilline, on a tout d'abord préparé de l'alcool divanillique en procédant comme suit (on aurait pu procéder selon l'exemple 8 de la demande de brevet EP 3 002 333). La divanilline purifiée (20 g) a été réduite avec du borohydrure de sodium (NaBH₄) afin de former l'alcool divanillinique. Pour cela, elle a été solubilisée dans de la soude à 0,5 M (180 mL ; quelques gouttes de solution 5M ont été opportunément ajoutées pour faciliter la solubilisation). Puis le NaBH₄ (3 g) a été incorporé et le mélange résultant a été maintenu sous agitation jusqu'à complète dissolution. Au bout d'une heure d'agitation, la réaction a été arrêtée par ajout, goutte à goutte, d'une solution aqueuse d'acide chlorhydrique (160 mL à 2M) jusqu'à atteindre un pH = 3. L'alcool divanillique a alors précipité. Il a été récupéré par filtration. Le produit ainsi récupéré a été séché à l'étuve. La synthèse a été répétée. Le rendement était à chaque fois d'environ 80 %.

On a confirmé l'obtention de l'alcool divanillique (DVA) par spectroscopie RMN :

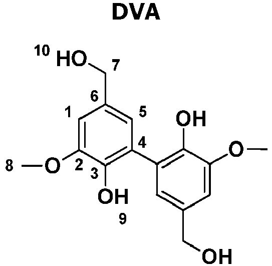

**RMN ¹H** (400 MHz, DMSO-d6, δ (ppm)) : δ 8.22 (s, H9), 6.88 (d, H1), 6.67 (d, H5), 5.01 (t, H10), 4.41 (d, H7), 3.82 (s, H8).

**RMN ¹³C** (400 MHz, DMSO-d6, δ (ppm)) : δ 147.94 (s, C3), 142.77 (s, C2), 133.08 (s, C6), 125.92 (s, C4), 121.83 (s, C5), 109.50 (s, C1), 63.38 (s, C7), 56.25 (s, C8). B) L'alcool divanillique obtenu a ensuite été époxydé avec de l'épichlorhydrine dans des conditions « différentes », pour l'obtention de mélanges différents de plusieurs composés pluriépoxydés.

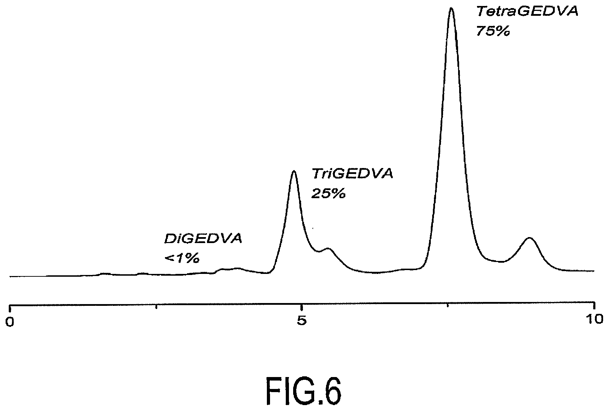

B1) On décrit précisément ci-après les conditions mises en œuvre pour l'obtention d'un mélange de 25 % de TriGEDVA et de 75 % de TétraGEDVA (% en masse). Dans un premier temps, l'alcool divanillique (20 g) a été mis en présence d'épichlorhydrine (100 mL) et de bromure de tétrabutylammonium (TEBAC) (2 g). Le TEBAC est un agent de transfert de phase qui permet au phénol de réagir avec l'épichlorhydrine, introduite en excès pour former un di-époxyde. Le mélange réactionnel a été laissé sous agitation à 80°C pendant 1 h 30 ; puis il a été refroidi à température ambiante. Par la suite, une solution aqueuse de soude (NaOH) (160 mL à 10 M : 10 NaOH eq./OH) a été ajoutée. L'ajout de la base a permis de fermer les époxydes ouverts mais également de déprotoner les alcools benzyliques qui, à leur tour, sont époxydés par substitution nucléophile avec l'épichlorhydrine. La solution a ensuite été agitée mécaniquement pendant 20 h dans un bain d'eau froide. A la fin de la réaction, du dichlorométhane (DCM) (300 mL) a été ajouté au milieu réactionnel pour faire précipiter les sels (NaCl). Les phases liquides ont été séparées du milieu réactionnel et les sels rincés avec 100 mL de DCM. Les phases liquides ont été rassemblées et la phase aqueuse a été extraite avec 2x50 mL de DCM. Les différentes phases organiques ont été rassemblées et lavées avec 100 mL d'eau. La phase organique a été concentrée en utilisant un évaporateur rotatif et l'épichlorhydrine a finalement été évaporée sous vide. Le rendement était quantitatif. La proportion de composés di-, tri- et tétraépoxydés a été quantifiée par HPLC (chromatographie en phase liquide à haute performance). L'appareil utilisé était un SpectraSYSTEM^{®}, monté d'une colonne Phenomenex 5µ C18 100A. Le détecteur utilisé était un système SpectraSYSTEM® UV2000 de Thermo Separation Products. Les analyses ont été réalisées avec un éluant composé d'acétonitrile et d'eau en proportion isocratique 50/50. La figure 6 annexée montre le chromatographe obtenu.

B2) On a reproduit (en tous points) la procédure décrite au point B1) ci-dessus mais avec ajout d'une solution aqueuse de NaOH (50 mL à 5 M) et avec agitation mécanique pendant seulement 1 h. On a alors obtenu un mélange de 80 % de DiGEDVA, de 15 % de TriGEDVA et de 5 % de TétraGEDVA (% en masse).

B3) On a reproduit (en tous points) la procédure décrite au point B1) ci-dessus mais avec ajout d'une solution aqueuse de NaOH (50 mL à 5 M) et avec agitation mécanique pendant seulement 8 h. On a alors obtenu un mélange de 35 % de DiGEDVA, 50 % de TriGEDVA et 15 % de TétraGEDVA (% en masse).

Pour obtenir, isolément, les différents composés pluriépoxydés (di-, tri- et tétraépoxydés, éléments constitutifs de résines époxy (pris isolément ou en mélange)), une étape de purification par chromatographie flash ou instantanée, sur un appareil Grace Reveleris^{®}, équipé d'une cartouche de sillice et d'un détecteur UV, a été mise œuvre sur les mélanges, en utilisant un gradient de solvant dichlorométhane/méthanol de 99/1 à 90/10 (en volume) pendant 30 minutes.

On a confirmé l'identité desdits composés pluriépoxydés par spectroscopie RMN :

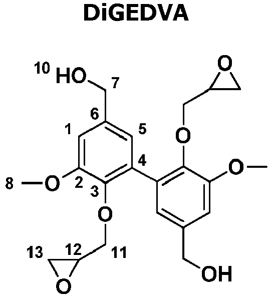

**RMN ¹H** (400MHz, DMSO-d6, δ (ppm)) : δ 7.0 (d, H₁), 6.71 (d, H₅), 5.16 (t, H₁₀), 4.47 (d, H₇), 3.88 (m, H₁₁), 3.83 (s, H₆), 3.74 (m, H_{11b}), 2.95 (m, H₁₂), 2.6 (t, H₁₃), 2.36 (t, H_{13b}).

**RMN ¹³C** (400MHz, DMSO-d6, δ (ppm)) : δ 152.33 (s, C₃), 144.47 (s, C₂), 138.26 (s, C₆), 132.59 (s, C₄), 120.86 (s, C₅), 110.79 (s, C₁), 74.22 (s, C₁₁), 63.14 (s, C₇), 56.18 (s, C₈), 50.53 (s, C₁₂), 43.97 (s, C₁₃).

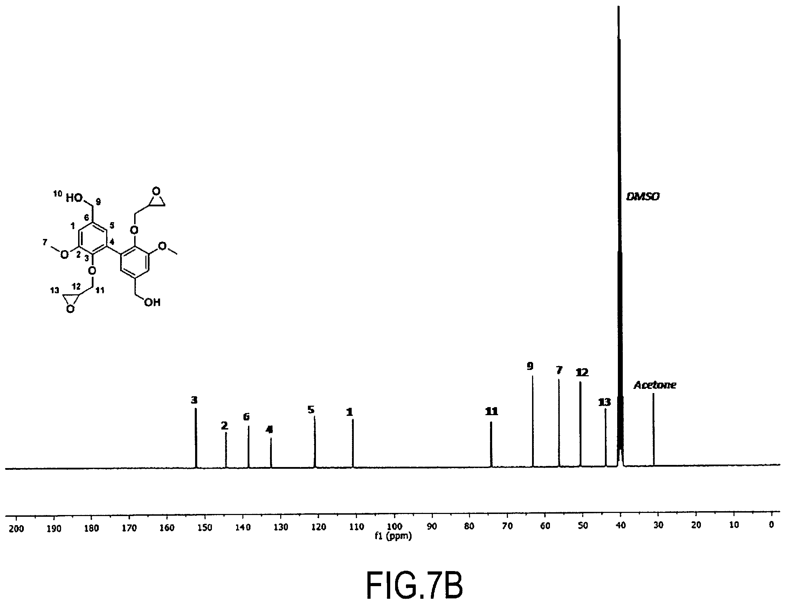

Les spectres sont respectivement montrés sur les figures 7A et 7B.

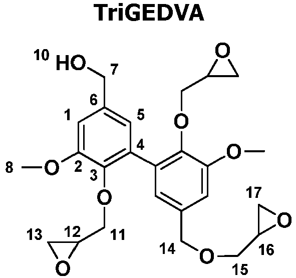

**RMN ¹H** (400MHz, DMSO-d6, δ (ppm)) : δ 7.01 (d, H₁), 6.75 (d, H₅), 5.18 (t, H₁₀), 4.47 (d, H₇ H₁₄), 3.92 (m, H₁₁), 3.84 (s, H₈), 3.76 (m, H_{11b}), 3.69 (m, H₁₅), 3.29 (m, H_{15b}), 3.14 (m, H₁₆), 2.97 (m, H₁₂), 2.72 (m, H₁₇), 2.6 (m, H₁₃), 2.5 (m, H_{17b}), 2.36 (m, H_{13b}).

**RMN ¹³C** (400MHz, DMSO-d6, δ (ppm)) : δ 152.02 (s, C₃), δ 151.89 (s, C₃), 144.38 (s, C_{2'}), 143.68 (s, C₂), 138.12 (s, C_{6'}), 133.39 (s, C₆), 132.06 (s, C_{4'}), 131.76 (s, C₄), 121.78 (s, C_{5'}), 120.26 (s, C₅), 111.55 (s, C_{1'}), 110.46 (s, C₁), 73.85 (s, C₁₄), 71.81 (s, C₁₅), 70.79 (s, C₁₁), 62.67 (s, C₇), 55.90 (s, C₈), 50.42 (s, C₁₂), 50.16 (s, C₁₆), 43.42 (s, C₁₃C₁₇).

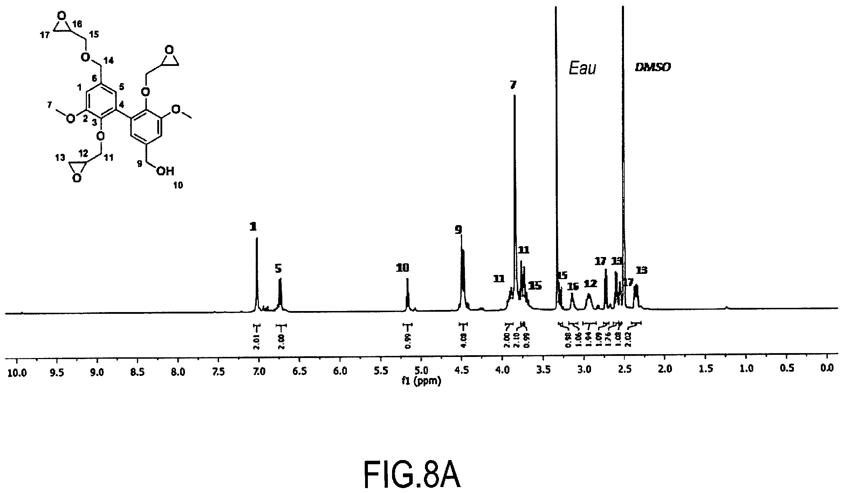

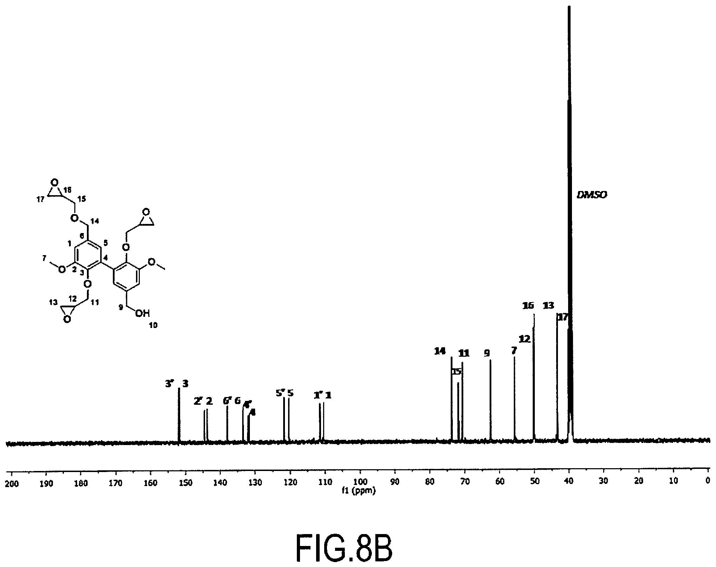

Les spectres sont respectivement montrés sur les figures 8A et 8B.

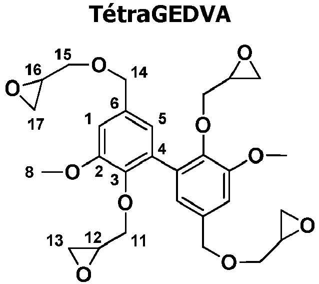

**RMN 1H** (400MHz, DMSO-d6, δ (ppm)): δ 7.02 (d, H1), 6.76 (d, H5), 4.50 (s, H14), 3.92 (m, H11), 3.86 (s, H8), 3.76 (m, H11b), 3.70 (m, H15), 3.28 (m, H15b), 3.14 (m, H16), 2.97 (m, H12), 2.73 (m, H17), 2.60 (m, H13), 2.55 (m, H17b), 2.35 (m, H13b). **RMN 13C** (400MHz, DMSO-d6, δ (ppm)): δ 152.10 (s, C3), 144.51 (s, C2), 133.51 (s, C6), 131.81 (s, C4), 121.83 (s, C5), 111.52 (s, C1), 73.77 (s, C14), 71.90 (s, C15), 63.14 (s, C11), 55.79 (s, C8), 50.30 (s, C12), 50.03 (s, C16), 43.44 (s, C13 C17).

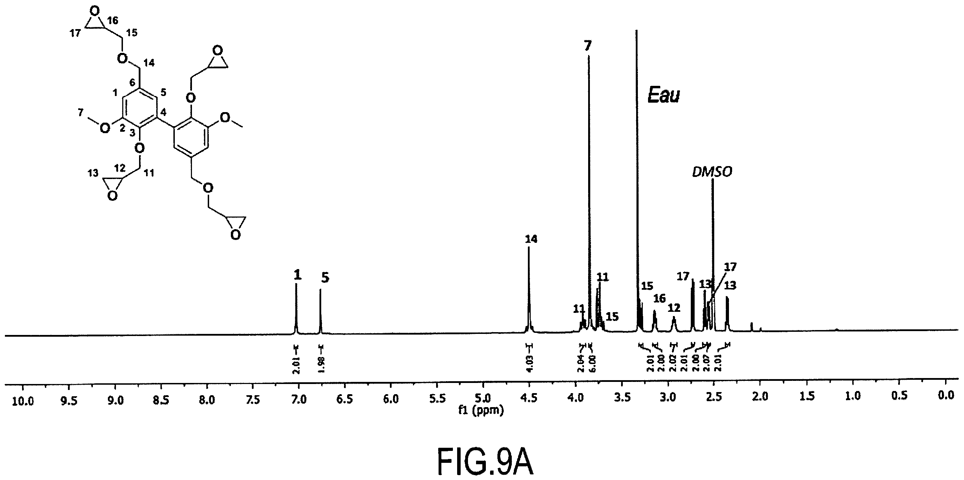

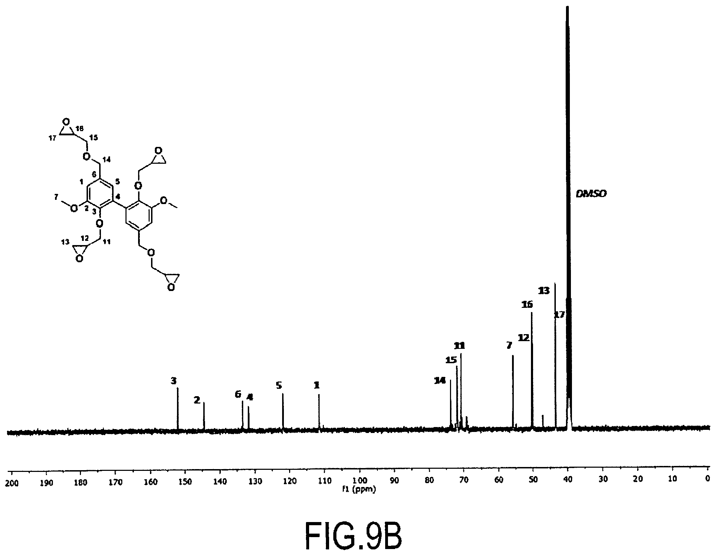

Les spectres sont respectivement montrés sur les figures 9A et 9B.

On a plus particulièrement mis en œuvre cette étape de purification sur le mélange obtenu à l'issue de l'étape B1 ci-dessus pour isoler le TétraGEDVA (résine époxy thermodurcissable) que l'on a testé avec des durcisseurs de nature différente.
- Les durcisseurs de type amine testés ont été :
   - le diaminodiphényl sulfone (Diamino Diphényl Sulfone = DDS), commercialisé par Sigma Aldrich. Celui-ci, durcisseur conventionnel, répond à la formule : 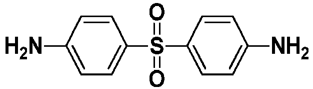 et
   - la 3,4-diméthoxydianiline de formule (I) (voir l'exemple 2 ci-dessus).

Le durcisseur a été utilisé, pour chaque test, en le ratio stœchiométrique : époxy/amine = 2/1.

Le polyépoxyde (résine époxy + durcisseur) a été généré, en petite quantité (quelques mg), lors de la mise en œuvre d'une calorimétrie différentielle à balayage (DSC). On a ainsi directement déterminé sa température de transition vitreuse (Tg).

Le taux de coke résiduel, après dégradation à 900°C, déterminé par TGA (ThermoGravimetric Analysis) (Char900), l'a été sur cette petite quantité générée lors de l'analyse DSC. Les résultats figurent dans le tableau 1 ci-après.

**Tableau 1**

| Propriétés | DGEBA/DDS | DGEBA/mDVAn | TétraGEDVA/mDVAn |
|---|---|---|---|
| Tg (°C) | 204 | 176 | 212 |
| Char900 (%) | 18 | 28 | 48 |

Les chiffres de ce tableau confirment l'intérêt des composés de l'invention.

## Revendications

1. Composé biphényle difonctionnel répondant à la formule (I) : 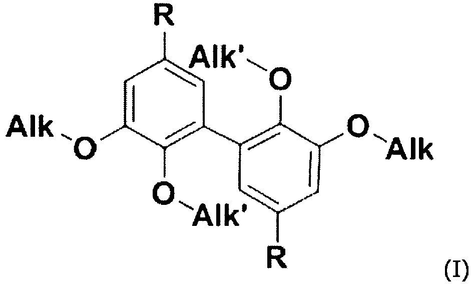 dans laquelle :
Alk est un groupe alkyle, linéaire ou ramifié, comportant de 1 à 6 atomes de carbone,
Alk' est un groupe alkyle, linéaire ou ramifié, comportant de 1 à 6 atomes de carbone, et
R est choisi parmi -CH₂-NH₂, -N=C=O et -NH₂;
étant entendu que le composé de formule (I) n'est pas la 3,4-diméthoxydianiline.

2. Composé biphényle selon la revendication 1, de formule (I) dans laquelle Alk est un groupe méthyle.

3. Composé biphényle selon la revendication 1 ou 2, de formule (I) dans laquelle Alk' est un groupe méthyle.

4. Composé biphényle selon l'une quelconque des revendications 1 à 3, qui consiste en :
- la 3,4-diméthoxydivanyllylamine, ou
- le 3,4-diméthoxydiphénylisocyanate.

5. Procédé de préparation d'un composé de formule (I) : 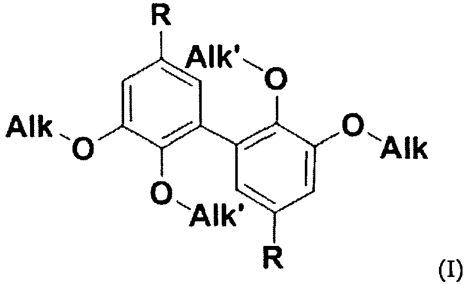 dans laquelle :
Alk est un groupe alkyle, linéaire ou ramifié, comportant de 1 à 6 atomes de carbone,
Alk' est un groupe alkyle, linéaire ou ramifié, comportant de 1 à 6 atomes de carbone, et
R est choisi parmi -CH₂-NH₂, -N=C=O et -NH₂ ;
qui comprend :
- la mise à disposition d'un produit choisi parmi la vanilline, les analogues de la vanilline présentant un groupe -O-(C₂-C₆)alkyle en position 3, les esters de la vanilline et les analogues desdits esters présentant un groupe -O-(C₂-C₆)alkyle en position 3 ;
- la dimérisation dudit produit pour l'obtention d'un dimère,
- le traitement dudit dimère obtenu pour la conversion de ses fonctions -OH phénoliques en fonctions alcoxy -OAlk' et, soit de ses fonctions aldéhydes en fonctions aminométhyle (-CH₂-NH₂), soit de ses fonctions esters en fonctions isocyanate (-N=C=O) ou amino (-NH₂).

6. Procédé selon la revendication 5, qui comprend la mise à disposition de vanilline d'origine naturelle ou d'un ester de vanilline obtenu à partir de vanilline d'origine naturelle.

7. Procédé selon la revendication 5 ou 6, dans lequel ledit traitement comprend :
- la mise à disposition d'un produit choisi parmi la vanilline et les analogues de la vanilline présentant un groupe O-(C₂-C₆)alkyle en position 3,
- la dimérisation dudit produit pour l'obtention d'un dimère,
- successivement, l'alkylation des fonctions -OH phénoliques dudit dimère puis la conversion des fonctions aldéhyde dudit dimère alkylé en fonctions oxime ou la conversion des fonctions aldéhyde dudit dimère en fonctions oxime puis l'alkylation des fonctions -OH phénoliques dudit dimère avec fonctions oxime, pour l'obtention d'une oxime divanillique alkylée ; avantageusement l'alkylation des fonctions -OH phénoliques dudit dimère puis la conversion des fonctions aldéhyde dudit dimère alkylé en fonctions oxime ; et
- la réduction de ladite oxime divanillique alkylée pour l'obtention d'une amine divanillique alkylée répondant à la formule (I) dans laquelle R = -CH₂-NH₂.

8. Procédé selon la revendication 5 ou 6, dans lequel ledit traitement comprend :
- la mise à disposition d'un produit choisi parmi les esters de la vanilline et les analogues desdits esters présentant un groupe -O-(C₂-C₆)alkyle en position 3 ;
- la dimérisation dudit produit pour l'obtention d'un dimère,
- successivement, la saponification dudit dimère pour obtenir un acide divanillique et l'alkylation des fonctions -OH phénoliques dudit acide divanillique ou l'alkylation des fonctions -OH phénoliques dudit dimère pour obtenir un ester divanillique alkylé et la saponification dudit ester divanillique alkylé, pour obtenir un acide divanillique alkylé ; avantageusement la saponification dudit dimère et l'alkylation des fonctions -OH de l'acide divanillique obtenu ;
- l'acylation dudit acide divanillique alkylé pour obtenir un diazoture d'acyle alkylé ; et
- la mise en œuvre d'un réarrangement de Curtius sur ledit diazoture d'acyle alkylé pour l'obtention d'un dialcoxydiphénylisocyanate répondant à la formule (I) dans laquelle R = -N=C=O ; et
- éventuellement, en sus, l'hydrolyse dudit dialcoxydiphényl isocyanate pour l'obtention d'une dianiline alkylée de formule (I) dans laquelle R = -NH₂.

9. Intermédiaire, utile à la préparation d'un composé de formule (I), selon l'une quelconque des revendications 1 à 4 et/ou préparé selon le procédé de l'une quelconque des revendications 5 à 8, choisi parmi :
- les 3,4-di(C₁-C₆)alcoxydivanillique oxime, à l'exception de la 3,4-diméthoxydivanillique oxime, et
- les 3,4-di(C₁-C₆)alcoxydiphényazoture d'acyle, tout particulièrement le 3,4-diméthoxydiphénylazoture d'acyle.

10. Utilisation d'au moins un composé de formule (I) : 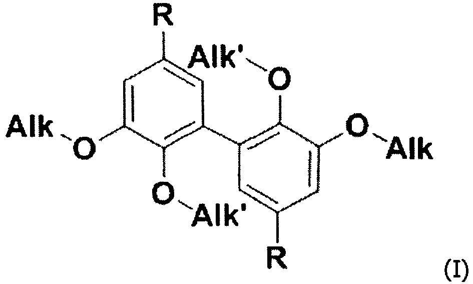 dans laquelle :
Alk est un groupe alkyle, linéaire ou ramifié, comportant de 1 à 6 atomes de carbone,
Alk' est un groupe alkyle, linéaire ou ramifié, comportant de 1 à 6 atomes de carbone, et
R est choisi parmi -CH₂-NH₂, -N=C=O et -NH₂ ;
à titre de durcisseur d'une résine thermodurcissable, notamment choisie parmi les résines époxy, les résines polycarbonates et les résines polyacide carboxylique pour l'obtention, respectivement, de polyépoxydes, de poly(hydroxy)uréthanes et de polyamides, et les résines polyols et les résines polyamines, pour l'obtention, respectivement, de polyuréthanes et de polyamides.

11. Résine thermodurcie, obtenue par traitement thermique, en présence d'au moins un durcisseur choisi parmi les composés de formule (I) : 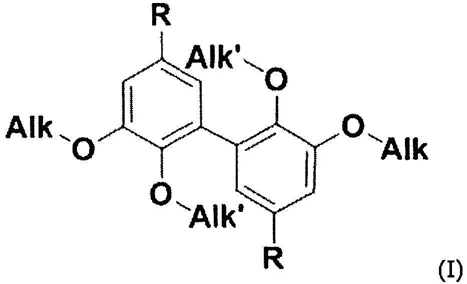 dans laquelle :
Alk est un groupe alkyle, linéaire ou ramifié, comportant de 1 à 6 atomes de carbone,
Alk' est un groupe alkyle, linéaire ou ramifié, comportant de 1 à 6 atomes de carbone, et
R est choisi parmi -CH₂-NH₂, -N=C=O et -NH₂ ;
d'une résine thermodurcissable, notamment choisie parmi les résines époxy, les résines polycarbonate, les résines polyacide carboxylique, ladite résine thermodurcie étant alors, respectivement, de type polyépoxyde, poly(hydroxy)uréthane ou polyamide, et les résines polyol et les résines polyamide, ladite résine thermodurcie étant alors, respectivement, de type polyol ou polyamide.

12. Résine thermodurcie, selon la revendication 11, de type polyépoxyde, obtenue par traitement thermique, en présence d'au moins un durcisseur choisi parmi les composés de formule (I) tels que définis à la revendication 11, dans laquelle R = -CH₂-NH₂ ou -NH₂, d'une résine époxy thermodurcissable renfermant au moins un composé biphényle pluriépoxydé choisi parmi :
- le diglycidyl éther de bisphénol, monomère ou oligomère,
- le diglycidyl éther d'alcool divanillique (DiGEDVA),
- le triglycidyl éther d'alcool divanillique (TriGEDVA),
- le tétraglycidyl éther d'alcool divanillique (TetraGEDVA), et
- les mélanges d'au moins deux desdits glycidyl éthers d'alcool divanillique.

13. Utilisation selon la revendication 10, ou résine thermodurcissable selon la revendication 11 ou 12, où dans la formule (I) Alk est un groupe méthyle.

14. Utilisation selon la revendication 10, ou résine thermodurcissable selon la revendication 11 ou 12, où dans la formule (I) Alk' est un groupe méthyle.

15. Utilisation selon la revendication 10, ou résine thermodurcissable selon la revendication 11 ou 12, où le composé de formule (I) est la 3,4-diméthoxydivanyllylamine, la 3,4-diméthoxydianiline ou le 3,4-diméthoxydiphénylisocyanate.

## Patentansprüche

1. Difunktionelle Biphenylverbindung mit der Formel (I): 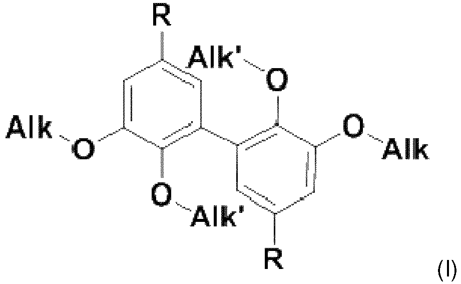 wobei:
Alk eine lineare oder verzweigte Alkylgruppe ist, die 1 bis 6 Kohlenstoffatome beinhaltet,
Alk' eine lineare oder verzweigte Alkylgruppe ist, die 1 bis 6 Kohlenstoffatome beinhaltet, und
R aus -CH₂-NH₂, -N=C=O und -NH₂ ausgewählt ist,
vorausgesetzt, dass die Verbindung der Formel (I) kein 3,4-Dimethoxydianilin ist.

2. Biphenylverbindung nach Anspruch 1, der Formel (I), wobei Alk eine Methylgruppe ist.

3. Biphenylverbindung nach Anspruch 1 oder 2, der Formel (I), wobei Alk' eine Methylgruppe ist.

4. Biphenylverbindung nach einem der Ansprüche 1 bis 3, bestehend aus:
- 3,4-Dimethoxydivanyllylamin, oder
- 3,4-Dimethoxydiphenylisocyanat.

5. Verfahren zur Herstellung einer Verbindung der Formel (I): 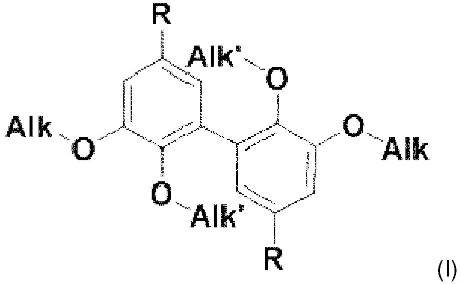 wobei:
Alk eine lineare oder verzweigte Alkylgruppe ist, die 1 bis 6 Kohlenstoffatome beinhaltet,
Alk' eine lineare oder verzweigte Alkylgruppe ist, die 1 bis 6 Kohlenstoffatome beinhaltet, und
R aus -CH₂-NH₂, -N=C=O und -NH₂ ausgewählt ist,
und umfasst:
- Bereitstellung eines Produktes ausgewählt aus Vanillin, Vanillinanaloga, die eine -O-(C₂-C₆)Alkylgruppe an Position 3 aufweisen, Vanillinestern und Analoga dieser Ester, die eine - O-(C₂-C₆)Alkylgruppe an Position 3 aufweisen,
- Dimerisierung des Produktes für den Erhalt eines Dimers,
- Behandlung des erhaltenen Dimers für die Umwandlung seiner phenolischen -OH-Funktionen in Alkoxy-OAlk'-Funktionen und entweder seiner Aldehydfunktionen in Aminomethylfunktionen (-CH₂-NH₂) oder seiner Esterfunktionen in Isocyanatfunktionen (-N=C=O) oder Aminofunktionen (-NH₂).

6. Verfahren nach Anspruch 5, das die Bereitstellung des Vanillins natürlichen Ursprungs oder eines Vanillinesters umfasst, der aus dem Vanillin natürlichen Ursprungs erhalten wird.

7. Verfahren nach Anspruch 5 oder 6, wobei die Behandlung umfasst:
- Bereitstellung eines Produktes, ausgewählt aus Vanillin und Vanillinanaloga, die eine O-(C₂-C₆)Alkylgruppe an Position 3 aufweisen,
- Dimerisierung des Produktes für den Erhalt eines Dimers,
- sukzessive Alkylierung der phenolischen -OH-Funktionen des Dimers, dann Umwandlung der Aldehydfunktionen des alkylierten Dimers in Oximfunktionen oder Umwandlung der Aldehydfunktionen des Dimers in Oximfunktionen, dann Alkylierung der phenolischen -OH-Funktionen des Dimers mit Oximfunktionen, für den Erhalt eines alkylierten Divanillinoxims, vorteilhafterweise Alkylierung der phenolischen -OH-Funktionen des Dimers, dann Umwandlung der Aldehydfunktionen des alkylierten Dimers in Oximfunktionen, und
- Reduzierung des alkylierten Divanillinoxims für den Erhalt eines alkylierten Divanillinamins mit der Formel (I), wobei R = -CH₂-NH₂.

8. Verfahren nach Anspruch 5 oder 6, wobei die Behandlung umfasst:
- Bereitstellung eines Produktes ausgewählt aus Vanillinestern und Analoga der Ester, aufweisend eine -O-(C₂-C₆)Alkylgruppe an Position 3,
- Dimerisierung des Produktes für den Erhalt eines Dimers,
- sukzessive Verseifung des Dimers, um eine Divanillinsäure zu erhalten, und Alkylierung der phenolischen -OH-Funktionen der Divanillinsäure oder Alkylierung der phenolischen -OH-Funktionen des Dimers, um einen alkylierten Divanillinester zu erhalten, und Verseifung des alkylierten Divanillinesters, um eine alkylierte Divanillinsäure zu erhalten, vorteilhafterweise Verseifung des Dimers und Alkylierung der -OH-Funktionen der erhaltenen Divanillinsäure,
- Acylierung der alkylierten Divanillinsäure, um ein alkyliertes Acyldiazid zu erhalten, und
- Umsetzung einer Curtius-Umordnung auf dem alkylierten Acyldiazid für den Erhalt eines Dialcoxydiphenylisocyanats mit der Formel (I), wobei R = -N=C=O, und
- gegebenenfalls zusätzlich Hydrolyse des Dialcoxydiphenylisocyanats für den Erhalt eines alkylierten Dianilins der Formel (I), wobei R = -NH₂.

9. Zwischenprodukt, nützlich für die Herstellung einer Verbindung der Formel (I), nach einem der Ansprüche 1 bis 4 und/oder hergestellt gemäß dem Verfahren nach einem der Ansprüche 5 bis 8, ausgewählt aus:
- 3,4-di(C₁-C₆)Alkoxydivanillinoxim, mit Ausnahme von 3,4-Dimethoxydivanillinoxim, und
- 3,4-di(C₁-C₆)Alkoxydiphenylacyldiazid, insbesondere 3,4-Dimethoxydiphenylacyldiazid.

10. Verwendung von mindestens einer Verbindung der Formel (I): 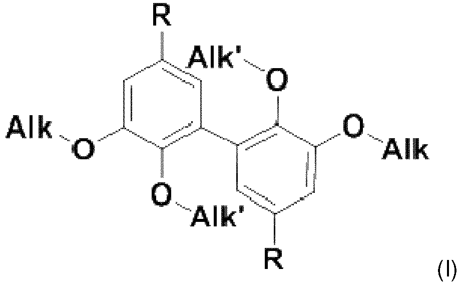 wobei:
Alk eine lineare oder verzweigte Alkylgruppe ist, die 1 bis 6 Kohlenstoffatome beinhaltet,
Alk' eine lineare oder verzweigte Alkylgruppe ist, die 1 bis 6 Kohlenstoffatome beinhaltet, und
R aus -CH₂-NH₂, -N=C=O und -NH₂ ausgewählt ist,
als Härtungsmittel eines wärmehärtbaren Harzes, insbesondere ausgewählt aus Epoxidharzen, Polycarbonatharzen und polyvalenten Carbonsäureharzen jeweils für den Erhalt von Polyepoxiden, Poly(hydroxy)urethanen und Polyamiden, und Polyolharzen und Polyaminharzen jeweils für den Erhalt von Polyurethanen und Polyamiden.

11. Wärmegehärtetes Harz, erhalten durch Wärmebehandlung, in der Gegenwart von mindestens einem Härtungsmittel ausgewählt aus den Verbindungen der Formel (I): 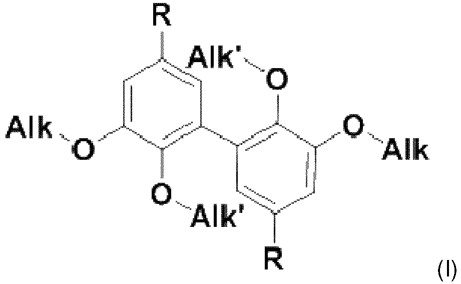 wobei:
Alk eine lineare oder verzweigte Alkylgruppe ist, die 1 bis 6 Kohlenstoffatome beinhaltet,
Alk' eine lineare oder verzweigte Alkylgruppe ist, die 1 bis 6 Kohlenstoffatome beinhaltet, und
R aus -CH₂-NH₂, -N=C=O und -NH₂ ausgewählt ist,
eines wärmehärtbaren Harzes, insbesondere ausgewählt aus Epoxidharzen, Polycarbonatharzen, polyvalenten Carbonsäureharzen, wobei das wärmegehärtete Harz jeweils des Typs Polyepoxid, Poly(hydroxy)urethan oder Polyamid ist, und Polyolharzen und Polyamidharzen, wobei das wärmegehärtete Harz jeweils des Typs Polyol oder Polyamid ist.

12. Wärmegehärtetes Harz nach Anspruch 11, des Typs Polyepoxid, erhalten durch Wärmebehandlung, in der Gegenwart von mindestens einem Härtungsmittel ausgewählt aus den Verbindungen der Formel (I) wie in Anspruch 11 definiert, wobei R = -CH₂-NH₂ oder - NH₂, eines wärmehärtbaren Epoxidharzes, das mindestens eine Pluriepoxid-Biphenylverbindung enthält, ausgewählt aus:
- dem Diglycidylether von Bisphenol, Monomer oder Oligomer,
- dem Diglycidylether von Divanillinalkohol (DiGEDVA),
- dem Triglycidylether von Divanillinalkohol (TriGEDVA),
- dem Tetraglycidylether von Divanillinalkohol (TetraGEDVA), und
- den Mischungen von mindestens zwei der Glycidylether von Divanillinalkohol.

13. Verwendung nach Anspruch 10, oder wärmehärtbares Harz nach Anspruch 11 oder 12, wobei in der Formel (I) Alk eine Methylgruppe ist.

14. Verwendung nach Anspruch 10, oder wärmehärtbares Harz nach Anspruch 11 oder 12, wobei in der Formel (I) Alk' eine Methylgruppe ist.

15. Verwendung nach Anspruch 10, oder wärmehärtbares Harz nach Anspruch 11 oder 12, wobei die Verbindung der Formel (I) 3,4-Dimethoxydivanyllylamin, 3,4-Dimethoxydianilin oder 3,4-Dimethoxydiphenylisocyanat ist.

## Claims

1. A difunctional biphenyl compound having the formula (I): 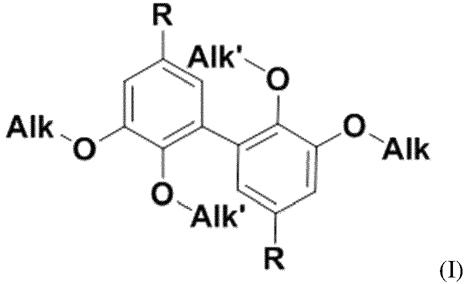 wherein:
Alk is a linear or branched alkyl group having from 1 to 6 carbon atoms,
Alk' is a linear or branched alkyl group having from 1 to 6 carbon atoms, and
R is selected from -CH₂-NH₂, -N=C=O and -NH₂;
provided that the compound of formula (I) is not 3,4-dimethoxydianiline.

2. The biphenyl compound of claim 1, of formula (I) wherein Alk is a methyl group.

3. The biphenyl compound of claim 1 or 2, of formula (I) wherein Alk' is a methyl group.

4. The biphenyl compound of any one of claims 1 to 3, which consists of:
- 3,4-dimethoxydivanyllylamine, or
- 3,4-dimethoxydiphenylisocyanate.

5. A process for preparing a compound of formula (I): 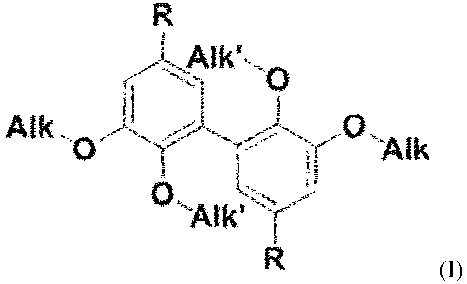 wherein:
Alk is a linear or branched alkyl group having from 1 to 6 carbon atoms,
Alk' is a linear or branched alkyl group having from 1 to 6 carbon atoms, and
R is selected from -CH₂-NH₂, -N=C=O and -NH₂;
which comprises:
- providing a product selected from vanillin, analogues of vanillin having an -O-(C₂-C₆)alkyl group in the 3-position, esters of vanillin and analogues of said esters having an -O-(C₂-C₆)alkyl group in the 3-position;
- dimerizing said product to obtain a dimer,
- treating said dimer obtained to convert its phenolic -OH functions to -OAlk' alkoxy functions and either its aldehyde functions to aminomethyl functions (-CH₂-NH₂) or its ester functions to isocyanate (-N=C=O) or amino (-NH₂) functions.

6. The process of claim 5, which comprises providing vanillin of natural origin or a vanillin ester obtained from vanillin of natural origin.

7. The process of claim 5 or 6, wherein said treating comprises:
- providing a product selected from vanillin and vanillin analogues having an O-(C₂-C₆)alkyl group in the 3-position,
- dimerizing said product to obtain a dimer,
- successively, alkylating the phenolic -OH functions of said dimer then converting the aldehyde functions of said alkylated dimer to oxime functions or converting the aldehyde functions of said dimer to oxime functions then alkylating the phenolic -OH functions of said dimer with oxime functions, in order to obtain an alkylated divanillyl oxime; advantageously alkylating the phenolic -OH functions of said dimer then converting the aldehyde functions of said alkylated dimer to oxime functions; and
- reducing said alkylated divanillyl oxime to obtain an alkylated divanillyl amine having the formula (I) wherein R = -CH₂-NH₂.

8. The process of claim 5 or 6, wherein said treating comprises:
- providing a product selected from vanillin esters and analogues of said esters having an -O-(C₂-C₆)alkyl group in the 3-position;
- dimerizing said product to obtain a dimer,
- successively, saponifying said dimer to obtain a divanillic acid and alkylating the phenolic
- OH functions of said divanillic acid or alkylating the phenolic -OH functions of said dimer to obtain an alkylated divanillyl ester and saponifying said alkylated divanillyl ester, to obtain an alkylated divanillic acid; advantageously saponifying said dimer and alkylating the -OH functions of the divanillic acid obtained;
- acylating said alkylated divanillic acid to obtain an alkylated acyl diazide; and
- carrying out a Curtius rearrangement on said alkylated acyl diazide to obtain a dialkoxydiphenylisocyanate having the formula (I) wherein R = -N=C=O; and
- optionally, in addition, hydrolyzing said dialkoxydiphenyl isocyanate to obtain an alkylated dianiline of formula (I) wherein R = -NH₂.

9. An intermediate, useful in the preparation of a compound of formula (I), as claimed in any one of claims 1 to 4 and/or prepared according to the process of any one of claims 5 to 8, selected from:
- 3,4-di(C₁₋C₆)alkoxydivanillyl oximes, with the exception of 3,4-dimethoxydivanillyl oxime, and
- acyl 3,4-di(C₁₋C₆)alkoxydiphenylazides, especially acyl 3,4-dimethoxydiphenylazide.

10. Use of at least one compound of formula (I): 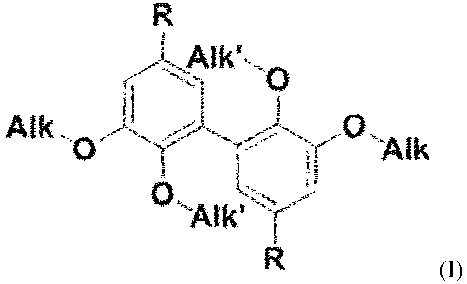 wherein:
Alk is a linear or branched alkyl group having from 1 to 6 carbon atoms,
Alk' is a linear or branched alkyl group having from 1 to 6 carbon atoms, and
R is selected from -CH₂-NH₂, -N=C=O and -NH₂;
as hardener of a thermosetting resin, in particular selected from epoxy resins, polycarbonate resins and polycarboxylic acid resins for obtaining, respectively, polyepoxides,
poly(hydroxy)urethanes and polyamides, and polyol resins and polyamine resins for obtaining, respectively, polyurethanes and polyamides.

11. A thermoset resin, obtained by heat treatment, in the presence of at least one hardener selected from the compounds of formula (I): 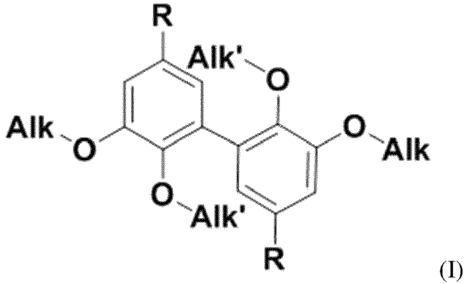 wherein:
Alk is a linear or branched alkyl group having from 1 to 6 carbon atoms,
Alk' is a linear or branched alkyl group having from 1 to 6 carbon atoms, and
R is selected from -CH₂-NH₂, -N=C=O and -NH₂;
of a thermosetting resin, in particular selected from epoxy resins, polycarbonate resins, polycarboxylic acid resins, said thermoset resin then being of the polyepoxide, poly(hydroxy)urethane or polyamide type, respectively, and polyol resins and polyamide resins, said thermoset resin then being of the polyol or polyamide type, respectively.

12. The thermoset resin of claim 11, of the polyepoxide type, obtained by heat treatment, in the presence of at least one hardener selected from the compounds of formula (I) as defined in claim 11, in which R = -CH₂-NH₂ or -NH₂, of a thermosetting epoxy resin containing at least one polyepoxide biphenyl compound selected from:
- diglycidyl ether of bisphenol, monomer or oligomer,
- diglycidyl ether of divanillyl alcohol (DiGEDVA),
- triglycidyl ether of divanillyl alcohol (TriGEDVA),
- tetraglycidyl ether of divanillyl alcohol (TetraGEDVA), and
- mixtures of at least two of said glycidyl ethers of divanillyl alcohol.

13. The use of claim 10, or the thermoset resin of claim 11 or 12, wherein in formula (I) Alk is a methyl group.

14. The use of claim 10, or the thermoset resin of claim 11 or 12, wherein in formula (I) Alk' is a methyl group.

15. The use of claim 10, or the thermoset resin of claim 11 or 12, wherein the compound of formula (I) is 3,4-dimethoxydivanyllylamine, 3,4-dimethoxydianiline or 3,4-dimethoxydiphenylisocyanate.
